(19) Europäisches Patentamt
European Patent Office
Office européen des brevets

(11) EP 1 104 547 B1

(12) EUROPÄISCHE PATENTSCHRIFT

(45) Veröffentlichungstag und Bekanntmachung des Hinweises auf die Patenterteilung:
**04.06.2003 Patentblatt 2003/23**

(21) Anmeldenummer: **99942859.2**

(22) Anmeldetag: **11.08.1999**

(51) Int Cl.$^7$: **G01N 33/92**

(86) Internationale Anmeldenummer:
**PCT/EP99/05911**

(87) Internationale Veröffentlichungsnummer:
**WO 00/010014 (24.02.2000 Gazette 2000/08)**

(54) **IN VITRO VERFAHREN ZUR ERKENNUNG UND DIAGNOSTIK AKUTER KORONARER SYNDROME**

IN-VITRO METHOD FOR DETECTING AND DIAGNOSING ACUTE CORONARY SYNDROMES

PROCEDE IN VITRO POUR DEPISTER ET DIAGNOSTIQUER DES SYNDROMES CORONARIENS AIGUS

(84) Benannte Vertragsstaaten:
**AT BE CH CY DE DK ES FI FR GB GR IE IT LI LU MC NL PT SE**

(30) Priorität: **12.08.1998 DE 19836617**

(43) Veröffentlichungstag der Anmeldung:
**06.06.2001 Patentblatt 2001/23**

(73) Patentinhaber:
- **Frei, Ulrich**
  **10557 Berlin (DE)**
- **Danne, Oliver**
  **14476 Seeburg (DE)**
- **Zschunke, Adolf**
  **12527 Berlin (DE)**
- **Mügge, Clemens**
  **13187 Berlin (DE)**

(72) Erfinder:
- **Frei, Ulrich**
  **10557 Berlin (DE)**
- **Danne, Oliver**
  **14476 Seeburg (DE)**
- **Zschunke, Adolf**
  **12527 Berlin (DE)**
- **Mügge, Clemens**
  **13187 Berlin (DE)**

(74) Vertreter: **Liesegang, Eva et al**
**Forrester & Boehmert,**
**Pettenkoferstrasse 20-22**
**80336 München (DE)**

(56) Entgegenhaltungen:
- **MEDLINE, Washington DC USA; abstract no. 88118997, abstract XP002124744 in der Anmeldung erwähnt & P.B. CORR ET AL.: "Lysophosphoglycerides and ventricular fibrillation early after onset of ischemia." JOURNAL OF MOLECULAR AND CELLULAR CARDIOLOGY, Bd. 19, Nr. supplement 5, 1. Oktober 1987 (1987-10-01), Seiten 45-53, St. Louis MI USA**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 104 547 B1

## Beschreibung

Die Erfindung betrifft ein in vitro Verfahren zur Erkennung und Diagnostik akuter koronarer Syndrome, insbesondere des akuten Myokardinfarktes (AMI), beim Menschen.

Akute koronare Syndrome umfassen die Krankheitsbilder des akuten Myokardinfarktes (AMI) und der instabilen Angina pectoris, welche durch die WHO-Klassifikation des AMI und die Braunwald-Klassifikation der instabilen Angina pectoris definiert werden (Gillum R.F. et al. 1984, Am Heart J 108: 150-158; Braunwald E. et al. 1994, Circulation 90: 613-622). Akute koronare Syndrome stellen einen häufigen und lebensbedrohlichen Erkrankungskomplex dar, bei dem die frühzeitige sichere Diagnose und Therapie entscheidend für das Überleben des Patienten sein kann. Dies trifft besonders auf den akuten Myokardinfarkt zu, bei dem eine Verzögerung der richtigen Diagnose und Therapie schwerwiegende Folgen für den Patienten hat. Bekannte Methoden zum Diagnostizieren eines Myokardinfarktes sind das Elektrokardiogramm und die Bestimmung verschiedener Labor-Marker. Das Elektrokardiogramm und die bisher bekannten Labor-Marker haben in der Frühphase des akuten Myokardinfarktes eine zu geringe Sensitivität, um bei einer Mehrheit der Patienten die Diagnose stellen zu können. So liegt die Sensitivität von infarkt-typischen Veränderungen im EKG (ST-Hebungen) bei 46 % (Rude, R.E. et al. 1983, Am J Cardiol 52:936-942). Die Sensitivität der Labor-Marker Creatinkinase (CK)-Aktivität, CK-MB-Aktivität, CK-MB-Masse, der CK-MB-Isoformen, von Myoglobin und kardialen Troponinen liegt in den ersten 2 Stunden nach Schmerzbeginn zwischen 11-29 % (Mair, J. et al. 1994, Mitteilungen der Deutschen Gesellschaft für Klinische Chemie 25: 1-6). Die begrenzte diagnostische Brauchbarkeit der bekannten Verfahren in der Frühphase des akuten Myokardinfarktes verursacht eine Reihe von klinischen Problemen wie die Gefahr von Fehldiagnosen, die Durchführung nicht-indizierter Therapien und die Verzögerung lebensrettender Therapien.

Bei der instabilen Angina pectoris können Risikopatienten durch die Bestimmung der kardialen Troponine relativ zuverlässig erkannt werden, die langsame Freisetzungskinetik bedingt aber, daß es auch hier in der Frühphase zu falsch-negativen Befunden kommt. Dies ist problematisch, weil diese Patienten eine ähnlich schlechte Prognose wie Patienten mit AMI haben können und eine rasche und gezielte antiischämische Therapie benötigen. Es besteht daher ein erheblicher Bedarf an Verfahren, die eine zuverlässige und frühzeitige Erkennung akuter koronarer Syndrome, insbesondere des akuten Myokardinfarktes, ermöglichen.

Die Erfindung hat daher zur Aufgabe, eine Methode anzugeben, welche die frühzeitige Erkennung akuter koronarer Syndrome ermöglicht und somit die Diagnose und Therapie der erkrankten Patienten verbessert.

Diese Aufgabe wird durch ein Verfahren mit den Merkmalen von Anspruch 1 gelöst. Bei dem erfindungsgemäßen Verfahren zur Erkennung akuter koronarer Syndrome, insbesondere des akuten Myokardinfarktes, wird der Gehalt von Cholin, Cholin- und/oder Trimethylammonium-Derivaten, ausgewählt aus der Gruppe, die Phosphorylcholin, Plasmalogene und Lysoplasmenylcholin umfaßt, in Körperflüssigkeiten oder Körperbestandteilen, die einem Patienten entnommen wurden, bestimmt und bewertet.

Cholin, Cholin- und Trimethylammonium-Derivate, ausgewählt aus der Gruppe, die Phosphorylcholin, Plasmalogene und Lysoplasmenylcholin umfaßt, sind Moleküle des Lipidstoffwechsels und werden im folgenden zusammenfassend als "**CCTD** " bezeichnet. CCTD haben folgende chemische Formeln:

**Cholin:** 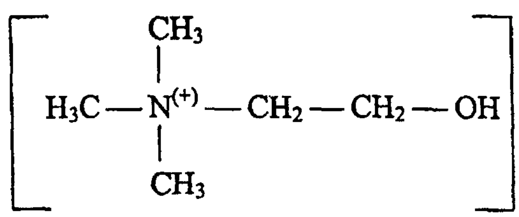 $OH^{(-)}$ **(Formel 1)**

Cholin-Derivate: 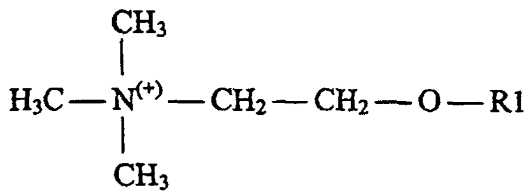 (Formel 2)

R1 = Rest 1

Trimethylammonium-Derivate: 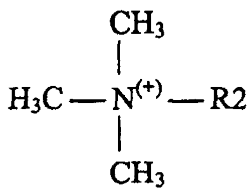 (Formel 3)

R2 = Rest 2

R1 und R2 stellen bestimmte chemische Substituenten dar, die die Substanzgruppe, die Phosphorylcholin, Plasmalogene und Lysoplasmenylcholin umfaßt, charakterisieren. Plasmalogene und Lysoplasmenylcholin enthalten in diesen Molekül-Anteilen eine Alkenyl-Gruppe.

Die Formel 1 zeigt, daß Cholin [2-Hydroxyethyl]trimethylammonium mit seinem Gegenion darstellt. Formel 2 zeigt die allgemeine chemische Formel für Cholin-Derivate und Formel 3 stellt die allgemeine Formel für Trimethylammonium-Derivate wie Phosphorylcholin und Plasmalogene wie Plasmenylcholin und Lysoplasmenylcholin dar. Die negative Ladung kann sich im gleichen Molekül oder in einem Gegenion befinden. Die Mehrzahl der CCTD sind entweder Bestandteile von Phospholipiden, welche Bausteine von biologischen Membranen darstellen oder eng mit dem Stoffwechsel der Phospholipide verbunden sind. Herzmuskelzellen sind besonders reich an Plasmenylcholinen, welche cholinhaltige Phospholipide darstellen, die eine charakteristische Alkenyl-Gruppe im Molekül besitzen. Plasmenylcholine sind zudem Bestandteile von Membranen der Mitochondrien, die einen bedeutsamen Teil der Myokardmasse ausmachen. Die Aktivierung von verschiedenen myokardialen Phospholipasen in der Frühphase des akuten Myokardinfarktes sowie die Störungen des Lipidstoffwechsels bei schwerer Myokardischämie führen entsprechend den Ergebnissen der Anmelder zu einer ausgeprägten Freisetzung von Cholin, Cholin-Derivaten und der chemisch verwandten Trimethylammonium-Derivate, wie Phosphorylcholin, Plasmalogenen oder Lysoplasmenylcholin, und verursachen eine Zunahme der Konzentration der CCTD in bestimmten Körperflüssigkeiten und Körperbestandteilen. Inwieweit nichtmyozytäre Elemente einschließlich der Endothel- und glatten Gefäßmuskelzellen daran beteiligt sind, ist nicht bekannt. Die chemische Verwandtheit von Cholin, Cholin-Derivaten und Trimethylammonium-Derivaten, ausgewählt aus der Gruppe, die Phosphorylcholin, Plasmalogene und Lysoplasmenylcholin umfaßt, und ihr gleichartiges Verhalten bei pathophysiologischen Prozessen, d.h. die sehr frühzeitige Freisetzung aus dem Herzen durch die ischämische Membrandestruktion, begründet die zusammenfassende Betrachtungsweise als CCTD.

Die möglichen Unterschiede der einzelnen CCTD treten vor der entscheidenden Gemeinsamkeit einer sehr raschem myokardialen Freisetzung bei akuten koronaren Syndromen in den Hintergrund, zumal diese Gemeinsamkeit entscheidend für das erfindungswesentliche Merkmal des Verfahrens bei der Frühdiagnostik in den ersten Stunden nach Schmerzbeginn ist.

Die diagnostische Nutzung der Freisetzung von Cholin, Cholin-Derivaten und Trimethylammonium-Derivaten, ausgewählt aus der Gruppe die Phosphorylcholin, Plasmalogene und Lysoplasmenylcholin umfaßt, zur Erkennung akuter koronarer Syndrome und des akuten Myokardinfarktes beim Menschen ist bisher noch nicht beschrieben worden. Bekannt ist lediglich, daß bei experimentellen Versuchsanordnungen in der Frühphase der Myokardischämie ein Anstieg von Lysophosphoglyceriden (z.B. Lysophosphatidylcholin) im Myokard sowie im venösen und lymphatischen Effluat zu beobachten ist (Corr P.B. et al. 1987, J Mol Cell Cardiol 19: 34-53; Snyder D.W. 1981, Am J Physiol 241: H700-H707; Akita H. et al. 1986, J Clin Invest 78: 271-280). Dabei wurde aber in keiner Publikation ein Verfahren für die Bewertung des Gehaltes von Cholin, Cholin-Derivaten und Trimethylammonium-Derivaten, ausgewählt aus der Gruppe die Phosphorylcholin, Plasmalogene und Lysoplasmenylcholin umfaßt, in Körperflüssigkeiten für die Diagnose

eines akuten koronaren Syndroms bzw. eines akuten Myokardinfarktes beim Menschen eingesetzt. Hintergrund der genannten Publikationen ist vielmehr die Beobachtung, daß Lysophosphatidylcholin offenbar proarrhythmische Effekte hat und daß Medikamente, die einen Anti-Lysophosphatidylcholin-Effekt haben, möglicherweise sinnvolle Therapeutika sein könnten.

Die Erfindung betrifft ein Verfahren zur Bestimmung von Cholin, Cholin- und/oder Trimethylammonium-Derivaten, wie Phosphorylcholin, Plasmalogenen und Lysoplasmenylcholin und oder bestimmten Reaktionsprodukten zur Frühdiagnostik akuter koronarer Syndrome. Dabei muß zwischen nicht-erfindungsgemäßen Diacyl-Phosphatidylcholinen und erfindungsgemäßen Plasmenylcholinen unterschieden werden. In der gleichen Weise müssen die nicht-erfindungsgemäßen Lysophosphatidylcholine mit einer Acyl-Gruppe im Molekül von den erfindungsgemäßen Lysoplasmenylcholinen mit einer Alkenyl-Gruppe im Molekül unterschieden werden. Unspezifische Bestimmungsverfahren, die die erfindungsgemäßen Substanzen nicht von anderen Phospholipiden oder ihren Bestandteilen differenzieren, weisen nicht die Merkmale des erfindungsgemäßen Verfahrens auf, die für die Frühdiagnostik von akuten koronaren Syndromen wesentlich sind, und können daher mit dem Verfahren nicht gleichgestellt werden.

So zeigen unspezifische Bestimmungsverfahren der Gesamtgruppe von Plasma-, Serum- oder Vollblut-Phospholipiden oder Lysophosphatidylcholinen nicht die Charakteristiken des erfindungsgemäßen Verfahrens bei der Frühdiagnostik akuter koronarer Syndrome. Diese in der Literatur beschriebenen unspezifischen Verfahren von Phospholipiden messen im Blut im wesentlichen hepatische Phospholipide, die eben gerade nicht die erfindungsgemäßen Plasmenylcholine und Lysoplasmenylcholine enthalten, sondern zu einem ganz überwiegenden Teil Cholin-Phospholipide ohne Alkenyl-Gruppe, z.B. Diacyl-Phosphatidylcholin, beinhalten. Da diese nicht erfindungsgemäßen Bestimmungsverfahren also gerade nicht die Substanzen spezifisch messen, die bei akuten koronaren Syndromen aus dem Herzen ins Blut freigesetzt werden, besitzen Sie auch nicht die Merkmale des erfindungsgemäßen Verfahrens. Mit den nicht-erfindungsgemäßen Bestimmungsverfahren von Phospholipiden werden beim Myokardinfarkt so entweder gar keine signifikanten Veränderungen oder sogar Konzentrationsverminderungen gemessen, was verdeutlicht, daß diese Verfahren eine Freisetzung von Substanzen aus dem Herzen mit einer entsprechenden Konzentrationszunahme im Blut nicht erfassen. Bei der Bestimmung der gesamten Gruppe von Lysolecithinen mißt man beispielsweise beim akuten Myokardinfarkt häufig eine Konzentrationsabnahme, da es im Rahmen der unspezifischen akuten Phase Reaktion zu einer verminderten Bildung verschiedener Acyl-Lysophosphatidylcholine durch eine Reduktion der Aktivität der Lecithin-Cholesterol-Acyl-Transferase (LCAT) kommt. Messungen mit unspezifischen Bestimmungsverfahren von Phospholipiden führen also häufig sogar zu entgegengesetzten Ergebnissen und Schlußfolgerungen, die für das erfindungsgemäße Verfahren nicht zutreffen.

Dabei läßt sich das erfindungsgemäße Verfahren mit verschiedenen analytischen Techniken durchführen, solange die erfindungsgemäßen Substanzen ausreichend spezifisch bestimmt werden können. So ist z.B. eine NMR-Spektroskopie, nach präanalytischer Zentrifugalultrafiltration der Probe und Entfernung protein-gebundener schlecht löslicher Diacyl-Phosphatidylcholine möglich. Ebenso können chromatographische, biochemische oder immunologische Bestimmungs-Techniken oder andere Methoden angewendet werden. Dabei kann das analytische Vorgehen in Anlehnung an publizierte Bestimmungsmethoden, wie z.B. biochemische bzw. enzymatisehe Methoden (Takayama, M. et al. 1977, Clin Chim Acta 79: 93-98), Hochleistungs-Flüssigkeits-Chromatographie (HPLC) (Brouwers, J.F.H. et al. 1998, J Lipid Res 39: 344-353; Potter, P. et al. 1983, J Neurochem 41: 188-94) sowie Gaschromatographie und/oder Massenspektrometrie (Myher, J.J. et al. 1989, Lipids 24: 396-407; Pomfret, A. et al. 1989, Anal Biochem 180: 85) oder immunologische Methoden (Smal, M.A. et al 1991, Lipids 26: 1130-1135; Baldo, B.A. et al 1991, Lipids 26: 1136-1139) vorgenommen werden. Übersichten zur Analytik von Phospholipiden wurden von Olsson publiziert (Olsson, N.U. et al.). Auf diese Veröffentlichungen wird Bezug genommen.

Bei erfindungsgemäßen biochemischen Methoden werden beispielsweise durch Hinzugabe von Reagenzien (z. B. Enzymen) chemische Reaktionen bzw. Reaktionsprodukte erzeugt, die dann die Detektion und Messung der Gesamtgruppe, einer Subgruppe oder Subspezies der CCTD ermöglichen (siehe Anwendungsbeispiele).

Bei erfindungsgemäßen immunologischen Methoden werden beispielsweise durch den Einsatz von immunologischen Reagenzien (z. B. Antikörpern), in der Regel in Kombination mit weiteren chemischen und/oder immunologischen Reagenzien, Reaktionen bzw. Reaktionsprodukte erzeugt, die dann die Detektion und Messung der Gesamtgruppe, einer Subgruppe oder Subspezies der CCTD ermöglichen (siehe Anwendungsbeispiele).

Bei erfindungsgemäßen chromatographischen Methoden wird beispielsweise, nach Vorbereitung der Probe, eine Stofftrennung durch die Verteilung zwischen einer ruhenden (stationären) und einer sich bewegenden (mobilen) Phase mit entsprechenden qualitativen und quantitativen Aussagen zu CCTD durchgeführt (siehe Anwendungsbeispiele).

Für jedes analytische Verfahren von CCTD und ihren Reaktionsprodukten sollte berücksichtigt werden, daß einige CCTD Einzelschichten, Doppelschichten, Membranen, Mizellen und/oder Vesikel bilden können, und daß diese Phänomene bedeutsam für die Analytik sein können.

Je nach gewählter Bestimmungstechnik sind quantitative Aussagen zu einzelnen, bestimmten Gruppen oder der gesamten Gruppe der erfindungsgemäß bestimmten Substanzen in dem Verfahren möglich. Die genannten Druckschriften werden durch Bezugnahme in die vorliegende Anmeldung aufgenommen.

Bei der kardialen Freisetzung von CCTD im Rahmen der ischämischen Membrandestruktion von Herzmuskelzellen sind bestimmte aktivierte Phospholipasen von Bedeutung. Diese Phospholipasen spalten Phospholipide auf, so daß neben CCTD auch einfache Reaktionsprodukte entstehen, die zwar keine Trimethylammonium-Gruppe tragen, aber in gleicher Weise freigesetzt werden. Da diese einfachen Reaktionsprodukte der CCTD Teil eines gemeinsamen Freisetzungsmechanismus sind, ist das Verfahren auch durch die Bestimmung solcher einfachen Reaktionsprodukte möglich. Neben der Phospholipase $A_2$, die u.a. zur Freisetzung von dem genannten Lysoplasmenylcholin beträgt, sind weitere wichtige Enzyme dieser Gruppe die Phospholipasen C und D, die die Esterbindung zwischen der Hydroxy-Gruppe am 3-sn-C-Atom des Glycerols und dem Phosphorylcholin (Phospholipase C) oder die Bindung zwischen der Phosphorylgruppe des 1,2-substituierten Glycerolphosphats und dem mit der Phosphorylgruppe veresterten Cholin angreift (Phospholipase D). Reaktionsprodukte der Aktivität dieser beiden Phospholipasen in Einwirkung auf die Plasmalogene sind demzufolge 1-O-Alk-1 '-enyl-2-substituiertes Glycerol (durch Phospholipase C) bzw. 1-O-Alk-1'-enyl-2-substituiertes Glycerolphosphat (durch Phospholipase D). Diese Reaktionsprodukte werden zusammen mit Cholin bzw. Phosphorylcholin frei, wenn die genannten Phospholipasen auf Plasmenylcholine einwirken. Somit kann das erfmdungsgemäße Verfahren zur Frühdiagnostik akuter koronarer Syndrome durch die Bestimmung von Cholin, Cholin- und/oder Trimethylammonium-Derivaten, ausgewählt aus der Gruppe, die Phosphorylcholin, Plasmalogene und Lysoplasmenylcholin umfaßt, und/oder deren Reaktionsprodukte, ausgewählt aus der Gruppe die 1-O-Alk-1'-enyl-2-substituiertes Glycerol und 1-O-Alk-1'-enyl-2-substituiertes Glycerolphosphat umfaßt, durchgeführt werden.

Neben der gesteigerten Aktivität von Phospholipasen können folgende weitere Vorgänge ursächlich für den Anstieg von CCTD und ihren Reaktionsprodukten sein: Verminderter Abbau und/oder gesteigerte Synthese von CCTD und ihren Reaktionsprodukten (mit entsprechenden Änderungen der dafür verantwortlichen Enzyme) und die Freisetzung aus zellulären Kompartimenten und Membranen durch sonstige Faktoren (mechanische Faktoren, Zellschwellung und andere Phänomene der myozytären Schädigung.)

Die Erfindung wird an folgenden Ausführungsbeispielen erläutert.

Fig. 1 zeigt ein $^1$H-NMR-Spektrum einer Blutprobe eines Patienten;

Fig. 2 zeigt vergrößerte Ausschnitte von zur Figur 1 analogen Spektren bei einem Patienten mit akutem Myokardinfarkt (A) und bei einem Patienten ohne akuten Myokardinfarkt (B). Analoge Erhöhungen der Fläche von $N^+(CH_3)_3$-Singuletts der CCTD können bei Patienten mit akutem Myokardinfarkt beobachtet werden.

Fig. 3. zeigt die Daten zur diagnostischen Wertigkeit von CCTD für die Diagnose eines akuten Myokardinfarktes im gesamten Prüfzeitraum;

Fig. 4. zeigt die Daten zur diagnostischen Wertigkeit von CCTD für die Diagnose eines akuten Myokardinfarktes in der Frühphase des AMI (0 bis 6 Stunden);

Fig. 5. zeigt die Daten zur diagnostischen Wertigkeit von CCTD für die Diagnose eines akuten Myokardinfarktes in der Frühphase des AMI (0 bis 3 Stunden); und

Fig. 6. zeigt die Daten zur diagnostischen Wertigkeit von CCTD für die Diagnose eines akuten Myokardinfarktes in der Spätphase des AMI (7 bis 35 Stunden).

**Auswahl und Entnahme einer geeigneten Probe einer Körperflüssigkeit**

Für die Durchführung des Verfahrens ist die Entnahme einer Probe einer Körperflüssigkeit notwendig. Dabei ist die diagnostische Wertigkeit des Verfahrens abhängig von der gewählten Körperflüssigkeit. Die Durchführung des Verfahrens ist beispielsweise durch die Untersuchung von Blutproben bzw. verarbeiteten Blutproben wie Plasma, Serum oder Vollblut möglich. Neben Blutproben können für das Verfahren auch andere Körperflüssigkeiten, wie z.B. Harn, untersucht werden. CCTD werden zu einem bestimmten Teil glomerulär filtriert und erscheinen daher z.T. auch im Harn. Dabei ist aber im Vergleich zu Untersuchungen in Blutproben mit einer zeitlichen Verzögerung von Konzentrationsänderungen zu rechnen, was ein Nachteil der Verwendung von Harn darstellt. Zudem läßt sich Harn nicht in jedem Fall so unkompliziert und rasch gewinnen wie Blut, was ebenfalls ein Nachteil darstellt. Vorteilhaft bei der Verwendung von Hamproben ist die Tatsache, daß häufig größere Probenmengen zur Verfügung stehen und daß durch die Messung einer Konzentration in einer Harnprobe, die über einen größeren Zeitraum produziert worden ist, auch pathophysiologische Vorgänge über ein größeres Zeitintervall erfaßt werden können. Dennoch ist aus den genannten Gründen für die Durchführung des Verfahrens die Verwendung von Blutproben bzw. verarbeiteten Blutproben sinnvoll. Die Bestimmung in anderen Körperbestandteilen und Körperflüssigkeiten, wie Gewebeproben, Lymphe oder Kapillarblut kann

sinnvoll sein. Bei der Probenabnahme sollten weitere Punkte berücksichtigt werden. Die exogene Zufuhr größerer Mengen von Cholin, Cholin-Derivaten oder Trimethylammonium-Derivaten vor der Probenabnahme sollte ausgeschlossen werden. Außerdem sollte für die Früherkennung akuter koronarer Syndrome die Probenabnahme auch so früh wie möglich erfolgen und gegebenenfalls periodischwiederholt werden.

**Bestimmung von CCTD in der Probe einer Körperflüssigkeit**

Das erfindungsgemäße Diagnostizier- und Bewertungsverfahren ist weitgehend unabhängig von der Bestimmungsmethode, solange eine ausreichende Spezifität für einzelne, eine Untergruppe oder alle der erfindungsgemäß bestimmten Substanzen besteht. Die erfindungsgemäß bestimmten Substanzen sind Cholin, Cholin- und/oder Trimethylammonium-Derivate, ausgewählt aus der Gruppe, die Phosphorylcholin, Plasmalogene und Lysoplasmenylcholin umfaßt, sowie bestimmte einfache Reaktionsprodukte der CCTD, ausgewählt aus der Gruppe, die 1-O-Alk-1'-enyl-2-substituiertes Glycerol und 1-O-Alk-1'-enyl-2-substituiertes Glycerolphosphat umfaßt. Als ein Ausführungsbeispiel wurde die $^1$H-NMR-Spektroskopie gewählt, die als primäres Meßverfahren auch zur Validierung verwendet werden kann. Neben Kemspinresonanz(NMR)-Methoden können auch biochemische, enzymatische, immunologische, klinisch-chemische, chromatographische, massenspektrometrische, elektrochemische, photometrische Methoden oder andere Methoden eingesetzt werden, solange mit hoher Selektivität kardial freigesetzte CCTD (bzw. deren einfache Reaktionsprodukte) bei Patienten mit Verdacht auf akute koronare Syndrome bestimmt werden.

Klinisch-chemische oder andere Methoden sowie Schnelltests zur Bestimmung von CCTD sollten vor ihrer Anwendung auf ihre analytische Qualität überprüft und validiert werden. Für die Anwendung zur Diagnostik von akuten koronaren Syndromen sollten dabei möglichst nur solche Methoden eingesetzt werden, die sich im Vergleich zur $^1$H-NMR-Spektroskopie diagnostisch als weitgehend gleichwertig oder überlegen erweisen. Die im folgenden genannten methodischen Ausführungen stellen Beispiele dar, d.h. qualitativ oder quantitativ andere Chemikalien, Lösungen, Reagenzien und Geräte können eingesetzt werden, wenn damit vergleichbare Ergebnisse erzielt werden. Grundsätzlich reicht für die Durchführung des Verfahrens die Anwendung *einer* validen Meßmethode für CCTD in *einer* geeigneten Körperflüssigkeit.

**Ausführungsbeispiel: Bestimmung von CCTD mittels $^1$H-NMR-Spektroskopie**

*Probenvorbereitung*

- Abnahme von 10 ml Blut
- Verarbeitung der Blutprobe je nach gewähltem Probenmaterial (Serum, Plasma oder Vollblut)
- Zentrifügalultrafiltration von 4 ml Probenmaterial über einen 10-kD-Filter (z.B. Ultrafree-4 B10; Millipore)
- Pipettieren von 600 µl Ultrafiltrat in ein 5-mm-NMR-Röhrchen (z.B. 527-PP-7, Willmad, Buena, USA)
- Pipettieren von 100 µl einer 3,5-mM-$d_4$-TSP-$D_2O$- Lösung als Konzentrationsstandard auf ein Probevolumen von 700 µl (TSP = Natrium-Salz der Trimethyl-Silylpropion-Säure)
- pH-Messung im 5-mm-NMR-Röhrchen (z.B. 3-mm-Minitrode, Hamilton) unmittelbar präoder postanalytisch

*Hochauflösende $^1$H NMR-Spektroskopie*

Die $^1$H-NMR-Spektren werden beispielsweise an einem 600 MHz-Spektrometer (z.B. Bruker AMX 600) unter folgenden Bedingungen aufgenommen:

- Single-Pulse-Technik
- Wasserunterdrückung mit der Vorsättigungstechnik
- 30-90° RF-Impuls
- 5-15 sec Pulsrepetitionszeit
- 64-128 Scans pro Probe

Die $^1$H-chemischen Verschiebungen werden auf $TSP_i$ intern bezogen. Die Charakterisierung von CH-, $CH_2$- und $CH_3$- Gruppen sowie weiterer protonentragender Gruppen bekannter Substanzen wird anhand publizierter $^1$H-Shift-Daten vorgenommen. Zusätzlich können Protonenresonanzen in unabhängigen Untersuchungen durch Addition von gereinigten Substanzen zugeordnet werden. Die Konzentrationsbestimmung einzelner Substanzen wird durch Bestimmung der Integrale der entsprechenden Protonenresonanzen und des $TSP_i$-Konzentrationsstandards unter Berücksichtigung der jeweiligen Protonenanzahl und des Verdünnungsfaktors vorgenommen. Dabei wird

die quantitative Auswertung der Spektren nach folgender Formel durchgeführt:

$$C_M = F_\mathrm{d} \frac{C_\mathrm{TSP}\, A_\mathrm{M}\, N_\mathrm{TSP}}{A_\mathrm{TSP}\, N_\mathrm{M}} \qquad \text{(Formel 4)}$$

$C_M$ = Konzentration des Metaboliten (M)
$C_\mathrm{TSP}$ = Konzentration des Konzentrationsstandards
$A_\mathrm{M}$ = Integral unterhalb des Peak of Interest (M)
$A_\mathrm{TSP}$ = Integral des Konzentrationsstandards
$F_\mathrm{d}$ = Verdünnungsfaktor durch die Hinzugabe des Konzentrationsstandards
$N_\mathrm{M}$ = Anzahl der M - Protonen
$N_\mathrm{TSP}$= Protonenanzahl des Konzentrationsstandards

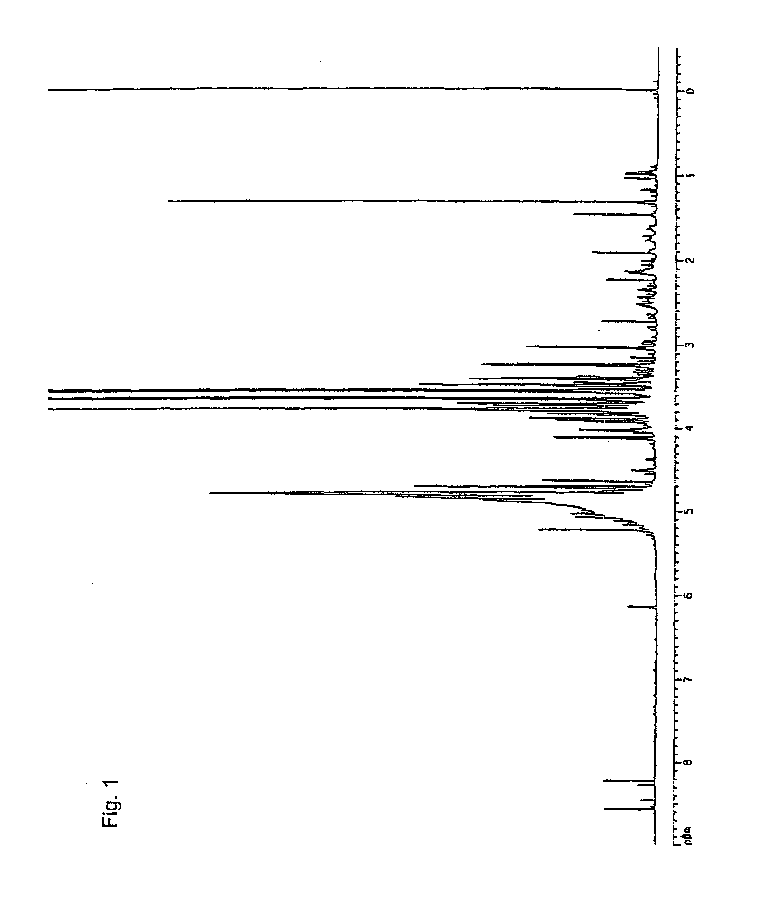

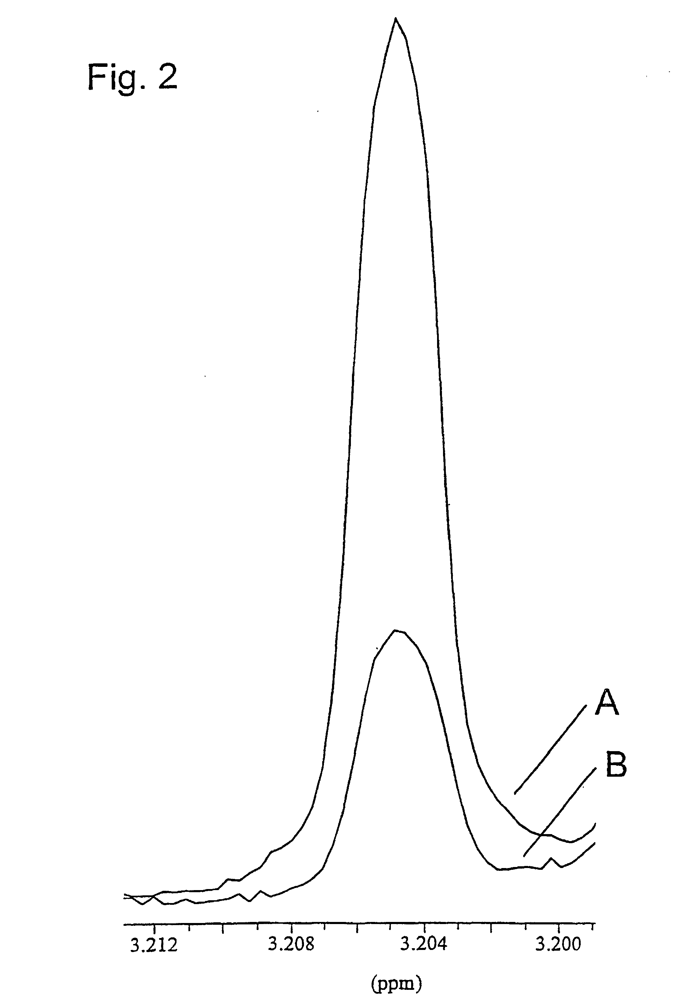

Die Methodik wurde von den Anmeldern für die Bestimmung von niedrig-molekularen Substanzen validiert und korreliert mit einem Korrelationskoeffizienten von r=0.998 mit enzymatischen Methoden. Fig. 1 zeigt ein $^1$H-NMR-Spektrum eines Patienten. Das typische Singulett der $N^+(CH_3)_3$-Gruppe von Cholin, Cholin-Derivaten und Trimethylammonium-Derivaten ist in den $^1$H-NMR-Spektren in der Regel in einem Bereich zwischen 2,5-4,5 ppm nachweisbar. Veränderungen der Protonen-Resonanzen sind durch verschiedene präanalytische Einflüsse, wie durch den pH möglich, die theoretisch zu Verschiebungen, Aufspaltungen und Überlagerungen führen können und daher bei der Auswertung berücksichtigt werden müssen. Es ist sinnvoll, die chemischen Verschiebungen der CCTD-Protonenresonanzen für die gewählten Untersuchungsbedingungen der NMR-Analytik in seperaten Untersuchungsreihen genau zu bestimmen. Das $N^+(CH_3)_3$-Singulett eignet sich besonders für die Quantifizierung, weil es neun äquivalente Protonen repräsentiert. Ein $N^+(CH_3)_3$-Smgulett mit dem typischen Befund beim akuten Myokardinfarkt ist in Fig. 2 dargestellt Die Konzentration von CCTD wird entsprechend der oben genannten Methode bestimmt. Die NMR-Spektroskopie bietet die Möglichkeit, z.B. durch Variation der Analytik und Auswertung, die Gesamtgruppe, eine Subgruppe oder Subspezies der CCTD zu bestimmen, während erfindungsgemäße immunologische oder biochemische Methoden häufig eine Subgruppe oder Subspezies der CCTD bestimmen. Weitere Ausführungsbeispiele des erfindungsgemäßen Verfahrens sind durch den Einsatz anderer CCTD-Bestimmungsmethoden als der NMR-Spektroskopie möglich. Bei erfindungsgemäßen biochemischen Methoden werden beispielsweise durch Hinzugabe von Reagenzien (z. B. Enzymen) chemische Reatkionen bzw. Reaktionsprodukte erzeugt, die dann die Detektion und Messung der Gesamtgruppe, einer Subgruppe oder Subspezies der CCTD ermöglichen. Eine solche Ausführung des erfindungsgemäßen Verfahrens kann z. B. durch eine Modifikation der von Takayama publizierten Methode durchgeführt werden (Takayama, M. et al., 1977, Clin. Chim. Acta 79:93-98). Die Bestimmung von Cholin erfolgt dabei über eine Oxidation von Cholin mittels einer Cholinoxidase, wobei als Reaktionsprodukte Betain und Wasserstoffperoxid ($H_20_2$) anfallen. Die Bildung von Wasserstoffperoxid ($H_20_2$) wird durch eine Farbreaktion, z. B. durch eine Kopplung von 4-Aminoantiporin und Phenol in Anwesenheit einer Peroxidase, nachgewiesen, und die Konzentration wird durch die Messung der Absorption bei 500 nm in einem kalibrierten UV-Spektrophotometer gegen die Blindprobe ermittelt.

Ein ähnliches, aber nicht-erfindungsgemäßes Verfahren kann auch für die Bestimmung der Gesamtgruppe von Cholin-Phospholipiden eingesetzt werden, wobei bei Verwendung einer unspezifischen Phospholipase D ohne Vorschalten eines weiteren Trennverfahrens die Methode nicht die erfindungswesentlichen Merkmale aufweist, da auch die große Gruppe von nicht-erfindungsgemäßen Diacyl-Phospholipiden mitgemessen wird. Die Spezifität der in der Analytik eingesetzten Phospholipase bzw. das Vorhandensein eines vorgeschalteten Trennverfahrens entscheidet über die Spezifität bzw. die Art der analysierten Substanzen. Wird eine unspezifische Phospholipase D eingesetzt und kein weiteres Trennverfahren vorgeschaltet, dann wird die unspezifische Gesamtgruppe von Cholin-Phospholipiden gemessen, so daß die erfindungswesentlichen Merkmale des Verfahrens nicht erfüllt sind. Wird eine spezifische Phospholipase D oder eine spezifische Lysophospholipase D eingesetzt, mit einer Substratspezifität für die erfindungsgemäßen Plasmenylcholine und/oder Lysoplasmenylcholine, ist eine erfindungsgemäße Ausführung des Verfahrens möglich, da erfindungsgemäße Substanzen dann spezifisch gemessen werden. Die Anwendung dieser Plasmalogen-spezifischen Phospholipasen bzw. Lysophospholipasen für die Analytik von CCTD ist bisher nicht publiziert worden, insbesondere nicht in der erfindungsgemäßen diagnostischen Anwendung bei akuten koronaren Syndromen. Wird vor dem Einsatz von Phospholipasen die Probe speziell aufbereitet (z.B. durch chromatographische Trennverfahren, Filtration oder Anreicherungsverfahren), so daß nicht-erfindungsgemäße Phospholipide, insbesondere Diacyl-Phospholipide, die aus der Leber stammen, entfernt wurden, spielt die Substratspezifität der verwendeten Phospholipase bzw. Lysophospholipase selbstverständlich keine so herausragende Rolle mehr, und die Spezifität des Verfahrens wird durch die vorgeschalteten Trennverfahren determiniert. Das Weglassen jeglicher Phospholipasen bzw. Lysophospholipasen führt im Ablauf der oben genannten Analytik zur Bestimmung von Cholin, was in der Anwendung bei der Diagnostik von akuten koronaren Syndromen ebenso eine erfindungsgemäße Durchführung des Verfahrens darstellt Die

Bestimmung von Cholin kann auch mit einer einfachen Fällungsreaktion mit anschließender Photometrie durchgeführt werden, wobei in jedem Fall bei diesen einfachen Ausführungen die Spezifität der Methode für erfindungsgemäße Substanzen genau überprüft werden sollte. Bei der erfindungsgemäßen biochemischen Bestimmung von Cholin oder anderen CCTD im Vollblut kann vor der quantitativen Bestimmung eine Hämolyse der Erythrozyten, z.B. durch Saponine durchgeführt werden.

Bei erfindungsgemäßen immunologischen Methoden werden beispielsweise durch den Einsatz von immunologischen Reagenzien (z. B. Antikörpern), in der Regel in Kombination mit weiteren chemischen und/oder immunologischen Reagenzien Reaktionen bzw. Reaktionsprodukte erzeugt, die dann die Detektion und Messung der Gesamtgruppe, einer Subgruppe oder Subspezies der CCTD ermöglichen. Die Durchführung dieser erfindungsgemäßen immunologischen Methoden kann in Anlehnung an die von Smal und Baldo publizierte Methodik vorgenommen werden (Smal, M.A. et al 1991, Lipids 26:1130-1135; Baldo, B.A. et al 1991, Lipids 26; 1136-1139). Auf diese Veröffentlichungen wird Bezug genommen. Dabei wird für die Entwicklung eines erfindungsgemäßen immunologischen Testverfahrens im ersten Schritt ein CCTD-Immungen hergestellt. Es werden synthetisierte oder hochgereinigte CCTD in stabile CCTD-Analoge überführt und an Proteine (z. B. methyliertes bovines Serumalbumin) gebunden. Daraufhin werden Tiere (z. B. Hasen oder Schafe) mit dem CCTD-Protein-Konjugat immunisiert und die gebildeten Anti-CCTD-Antikörper isoliert und gereinigt, z. B. unter Verwendung einer Affinitätschromatographie. Die Spezifität der Antikörper für CCTD und/oder eine Subgruppe oder Subspezies von CCTD sollte entsprechend überprüft werden. Die Anti-CCTD-Antikörper können schließlich mit verschiedenen immunologischen Techniken zur quantitativen Diagnostik eingesetzt werden.

Bei Verwendung eines Radioimmunoassays konkurrieren radioaktiv markierte Antigene (z. B. $^{125}$I-CCTD mit dem Antigen der Probe (CCTD der Probe) um eine im Unterschuß vorliegende Menge Anti-CCTD-Antikörper im Testansatz. Die Verdrängung der radioaktiv markierten CCTD von Anti-CCTD-Antikörpern steht in einer quantitativen Beziehung zur CCTD-Konzentration in der Probe, welche nach Erstellung einer Standardkurve und z. B. nach Fällung von an Antikörper gebundenem radioaktiven CCTD bestimmt werden kann.

Nach einem ähnlichen Prinzip kann ein CCTD-Chemilumineszenz-Immunoassay (CCTD-CELIA) zur Bestimmung von CCTD durchgeführt werden. CCTD und Luminol-markiertes Antigen konkurrieren um eine limitierte Menge Festphase-gebundener Antikörper. Das meßbare Lichtsignal ist umgekehrt proportional zur CCTD-Konzentration in der Patientenprobe.

Des weiteren kann die immunologische Bestimmung von CCTD auch durch ein CCTD-Fluoreszenz-Polarisations-Immunoassay durchgeführt werden. Dabei konkurrieren eine definierte Menge Fluorophor markierter CCTD mit den CCTD der Probe um eine im Unterschuß vorliegende Menge korrespondierender Anti-CCTD-Antikörper. Registriert wird die Beeinflussung der Fluorophor-markierten CCTD nach Antikörperbindung. Zur Messung angewendet wird die Technik der Fluoreszenz-Polarisation, bei der die Änderung des Winkels der polarisierten Fluoreszenzstrahlung, die das Fluorophor nach Anregung emittiert, gemessen wird. Liegen die Fluorophor-markierten CCTD an Antikörper gebunden vor, können sie sich wenig, in freier Form aber gut im Zeitraum zwischen Absorption und Emission von Strahlung drehen. Die Polarisation ist groß (Winkeländerung der emittierten Strahlung klein) bei niedriger CCTD-Konzentration und klein (Winkeländerung groß) bei hoher CCTD-Konzentration.

Die erfindungsgemäße immunologische Bestimmung von CCTD kann ebenfalls als Sandwich-Assay durchgeführt werden, wenn die zu bestimmenden CCTD, bzw. Subgruppen oder Subspezies von CCTD mindestens zwei verschiedene Epitope aufweisen oder sich durch eine spezifische Reaktion in eine Substanz mit mindestens zwei verschiedenen Epitopen überrühren läßt. In diesem Fall ist die Durchführung eines CCTD-enzyme linked immunoabsorbent assay (CCTD-ELISA) möglich. Der Anti-CCTD-Antikörper ist an eine feste Phase, z.B. eine Mikrotiterplattenvertiefüng, magnetische Partikel, Plastikperlen oder an eine Röhrchenwand gekoppelt. Wird die entsprechend vorbereitete Probe dazugegeben, binden CCTD der Probe an den Antikörper. Die Menge des gebundenen CCTD-Antigens wird durch die Zugabe eines markierten Zweitantikörpers ermittelt, der an das CCTD-Antigen unter Bildung eines Sandwich bindet. Je höher die Konzentration der gebundenen CCTD, desto größer ist die Menge des im Sandwich gebundenen, z.B. durch einen Enzym-markierten Zweitantikörper. Für einen bestimmten CCTD-Konzentrationsbereich besteht eine lineare Beziehung zwischen der CCTD-Konzentration und der Enzymaktivität.

Ein ähnliches erfindungsgemäßes Bestimmungsverfahren ist das Microbeadenzymimmunoassay von CCTD (CCTD-MEIA), bei dem der Anti-CCTD-Antikörper an Mikroperlchen gebunden ist. In einem ersten Schritt wird die Probe mit den Antikörper-Mikroperlchen inkubiert, dann wird eine alkalische Phosphatase(AP)-markierter Zweitantikörper hinzugegeben. Es kommt zur Ausbildung eines Sandwich am Mikroperichen. In einem weiteren Schritt wird ein Teil des Reaktionsgemisches auf eine Glasfasermatrix pipettiert, die eine hohe Affinität zu den Mikroperlchen hat und diese bindet. Nach Waschen der Glasfasermatrix mit Substratlösung und Entfernung von nicht gebundenem AP-markiertem Zweitantikörper kann die als Sandwich an den Mikroperlchen gebundene AP z.B. vom Substrat Methylumbelliferrylphosphat die Phosphatgruppe abspalten. Die Fluoreszenz des gebildeten Methylumbelliferon wird im Auflicht bei 448 nm gemessen. Die Sandwich-Techniken sind für kleine Moleküle mit nur einem Epitop ungeeignet und lassen sich nur bei CCTD anwenden, die ausgewählt sind aus der Gruppe von CCTD mit mindestens zwei Epitopen bzw. den CCTD, die sich durch eine spezifische chemische Reaktion in analoge Moleküle mit mindestens 2 Epitopen überführen

lassen.

Weitere Varianten und Spezifikationen immunologischer CCTD-Bestimmung können durchgeführt werden. Dazu zählen direkte immunologische Antigen-Bestimmungen, Bestimmungen als lösliche Immunkomplexe, indirekte Antigen-Bestimmungen, weitere Antigen- bzw. Antikörperbestimmungen durch Markierung eines Reaktionspartners, Liganden-Bindungsassays und weitere heterogene und homogene Immunoassays. Im Rahmen der immunologischen CCTD-Bestimmung können Trenntechniken wie Adsorption, Fällung, Immunfällung und/oder das Festphasenprinzip angewendet werden sowie verschiedene Markierungstechniken, wie radioaktiver Label (z.B. $^{125}I$), enzymatischer Label (z.B. alkalische Phosphatase, Meerrettichperoxidase, Glucose-6-Phosphat-Dehydrogenase), Fluoreszenz-Label oder Lumineszenz-Label (z.B.Chemilumineszenz, Biolumineszenz) angewendet werden. Entscheidendes Kriterium ist die spezifische Bestimmung von CCTD bzw. einer Subgruppe oder Subspezies von CCTD oder den genannten Reaktionsprodukten und die Anwendung zur Diagnostik akuter koronarer Syndrome.

Bei erfindungsgemäßen chromatographischen Methoden zur Bestimmung von CCTD wird beispielsweise, nach Vorbereitung der Probe, eine Stofftrennung durch die Verteilung zwischen einer ruhenden (stationären) und einer sich bewegenden (mobilen) Phase mit entsprechenden qualitativen und quantitativen Aussagen zu CCTD durchgeführt. Die erfindungsgemäße Bestimmung von CCTD kann dabei in Anlehnung an die Methode von Brouwers durchgeführt werden (Brouwers, J.F.H. et al., 1998, J Lipid Res 39: 344-353). Nach Präparation der Probe und Lipidextraktion werden zunächst Phosphatidylcholine von anderen Lipiden getrennt. Dies kann mittels hochauflösender Flüssigkeitschromatographie (HPLC) z.B. als Normalphasen-HPLC (NP-HPLC) durchgeführt werden. Nach entsprechender Trennung und Sammlung der Eluate wird eine Reverse-Phase Chromatographie (RP-HPLC) beispielsweise mit Acetonitril, Methanol, und Triethylamin als Lösungsmittel durchgeführt. Als Detektor-System eignet sich eine Light-Scattering-Detection. Durch die Untersuchung von Standardlösungen in Kombination mit Phosphorbestimmungen können Kalibrationskurven erstellt werden, so daß eine quantitative Bestimmung von CCTD und insbesondere auch die Bestimmung von bestimmten CCTD-Subspezies möglich ist.

Die chromatographischen und immunologischen Bestimmungsmethoden haben dabei im Vergleich mit der NMR-Methode den Vorteil, z.B. eine bestimmte Plasmenylcholin-Subspezies, mit einer definierten Alkenyl-Gruppe in der sn-1 Position und einer bestimmten Acyl-Gruppe in der sn-2 Position spezifisch zu bestimmen, bzw. von anderen ähnlichen Molekülen zu unterscheiden und damit die Organ-Spezifität des Verfahrens zu erhöhen.

Für den Fachmann ist es offensichtlich, daß zu den oben genannten Methoden ähnliche Bestimmungsverfahren für die erfindungsgemäße Bestimmung von CCTD-Reaktionsprodukten ebenso eingesetzt werden können.

Für die erfindungsgemäße Bestimmung von CCTD-Subspezies eignen sich besonders die Moleküle, bei denen die Anzahl falsch positiver Testergebnisse bei der Diagnostik akuter koronarer Syndrome gering bleibt. Unter anderen Subspezies sind dabei folgende CCTD-Subspezies einschließlich ihrer Reaktionsprodukte bedeutsam und können einzeln oder in beliebiger Kombination für die erfindungsgemäße Durchführung des Verfahrens bestimmt werden: 16:0-20:3 Plasmenylcholin, 16:0-18:3 Plasmenylcholin, 17:0-18:2 Plasmenylcholin, 15:0-18:2 Plasmenylcholin, 17:0-18:1 Plasmenylcholin, 17:0-18:3 Plasmenylcholin, 16:0-17:1 Plasmenylcholin, 14:0-18:2 Plasmenylcholin, 15: 0-18:1 Plasmenylcholin, 16:0 Lysoplasmenylcholin, 17:0 Lysoplasmenylcholin, 15:0 Lysoplasmenylcholin, 14:0 Lysoplasmenylcholin und die Reaktionsprodukte 16:0(Alk-1-enyl)-20:3 Glycerol, 16:0(Alk-1-enyl)-18:3 Glycerol, 17:0(Alk-1-enyl)-18:2 Glycerol, 15:0(Alk-1-enyl)-18:2 Glycerol, 17:0(Alk-1-enyl)-18:1 Glycerol, 17:0(Alk-1-enyl)-18:3 Glycerol, 16:0(Alk-1-enyl)-17:1 Glycerol, 14:0(Alk-1-enyl)-18:2 Glycerol, 15:0(Alk-1-enyl)-18:1 Glycerol, 16:0(Alk-1-enyl)-20:3 Glycerolphosphat, 16:0(Alk-1-enyl)-18:3 Glycerolphosphat, 17:0(Alk-1-enyl)-18:2 Glycerolphosphat, 15:0(Alk-1-enyl)-18:2 Glycerolphosphat, 17:0(Alk-1-enyl)-18:1 Glycerolphosphat, 17:0(Alk-1-enyl)-18:3 Glycerolphosphat, 16:0(Alk-1 -enyl)-17:1 Glycerolphosphat, 14:0(Alk-1-enyl)-18:2 Glycerolphosphat und 15:0(Alk-1-enyl)-18:1 Glycerolphosphat. Weitere CCTD-Subspezies und Reaktionsprodukte können für das erfindungsgemäße Verfahren ausgewählt werden, sofern mit deren Bestimmung die erfindungsgemäßen Merkmale erfüllt werden.

Bei Durchführung des Verfahrens mit der NMR-Spektroskopie können neben der Analyse von $N^+(CH_3)_3$-Singuletts auch andere Molekülanteile in die Analyse einbezogen werden. Desweiteren kann die NMR-Spektroskopie als ein, zwei- oder mehrdimensionale NMR-Spektroskopie oder mit anderen NMR-spektroskopischen Untersuchungstechniken u.a. auch in Kopplung z.B. an chromatographische Verfahren (beispielsweise als LC-NMR) durchgeführt werden.

Die Gemeinsamkeit der verschiedenen möglichen erfindungsgemäßen Bestimmungsmethoden und Techniken liegt darin, daß die Spezifität der Methode für CCTD hoch ist, so daß die erfindungswesentlichen Merkmale der Frühdiagnostik akuter koronarer Syndrome erfüllt sind. Für die selektive Bestimmung kardial freigesetzter CCTD mit einem erfindungsgemäßen Verfahren können dabei bestimmte Schritte der Probenvorbereitung wichtig sein. So werden beispielsweise in der beschriebenen Methodik durch die präanalytische Ultrafiltration nicht-erfindungsgemäße, Protein-gebundene hepatische Diacyl-Cholinphospholip'ide in der Menge reduziert bzw. je nach Filter auch vollständig eliminiert, während die freie lösliche Fraktion kardial freigesetzter CCTD einschließlich der Plasmalogene im Ultrafiltrat gemessen wird. Andere methodische Schritte der Reinigung und/oder Extraktion oder biochemischen Modifikation können angewendet werden und zu vergleichbaren Ergebnissen führen. Desweiteren kann das erfindungsgemäße

Verfahren so durchgeführt werden, daß eine, mehrere oder alle molekularen Subspezies von kardial freigesetzten CCTD bzw ihren Reaktionsprodukten analysiert werden.

Neben der Bestimmung von CCTD bietet die $^1$H-NMR-Spektroskopie die Möglichkeit, in demselben Untersuchungsgang weitere Metabolite, wie Creatin (Singulett bei 3,93 ppm) und Dimethylamin (Singulett bei 2,727 ppm), zu bestimmen, die ebenfalls für die Infarktdiagnostik von Bedeutung sind. Die Kombination der Auswertung mehrer Metabolite in einem Untersuchungsgang (z.B. CCTD, Creatin und Dimethylamin) wird hier als Pattem-Recognition (Muster-Erkennung) bezeichnet. Gegenüber der isolierten Konzentrationsbestimmung von CCTD verbessert die Auswertung im Pattern-Recognition Modus die diagnostische Aussagekraft der Methode für die Erkennung akuter koronarer Syndrome.

**Bewertung der Meßergebnisse von CCTD**

Die Bewertung der Meßergebnisse wird unter Berücksichtigung eines Grenzwertes vorgenommen. Der Grenzwert ist abhängig von der untersuchten Körperflüssigkeit und der gewählten Bestimmungsmethode und muß durch entsprechende Untersuchungen festgelegt werden. Liegt der bestimmte Meßwert für CCTD höher als der Grenzwert, liegt ein akuter Myokardinfarkt vor. Wenn der Meßwert niedriger als der Grenzwert ist, kann ein akuter Myokardinfarkt nahezu ausgeschlossen werden. Analoges gilt für die Diagnose der instabilen Angina pectoris, wobei die Grenzwerte durch die Untersuchung einer ausreichenden Anzahl von Patienten und in Hinblick auf die diagnostische Fragestellung festgestellt werden müssen. In der von uns durchgeführten Methodik der Konzentrationsbestimmung von CCTD liegt der Grenzwert je nach Auswertungsmodus zwischen 15-40 µmol/l. Die dargestellten klinischen Daten beziehen sich auf eine Auswertung mit einem Grenzwert von 22 µmol/l. Dabei muß der Grenzwert, wie bereits erwähnt, im einzelnen für die gewählte analytische Methodik, den Auswertungsmodus und die genaue Fragestellung bestimmt werden und kann z.B. bei der immunologischen oder chromatographischen Bestimmung von CCTD-Subspezies auch in einem anderen Meßbereich, z.B. im nanomolaren oder subnanomolaren Bereich liegen.

Bei der Durchführung der $^1$H-NMR-Spektroskopie kann zusätzlich zur isolierten Konzentrationsbestimmung von CCTD eine Auswertung im Pattem-Recognition-Modus vorgenommen werden (z.B. zusätzliche Bestimmung von Creatin und Dimethylamin). Bei der Auswertung im Pattern-Recognition-Modus wurde die Erhöhung von CCTD oder Creatin bei gleichzeitig normalen Konzentrationen von Dimethylamin für die Diagnose eines AMI herangezogen.

**Befunde bei Patienten**

Insgesamt wurden 20 Patienten und Probanden (8 f, 12 m) im Alter von 22-68 Jahren, teilweise periodisch, mit insgesamt 44 Proben untersucht. Weitere Proben wurden mit speziellen Fragestellungen untersucht. 10 Patienten hatten einen akuten Myokardinfarkt (4 Vorderwandinfarkte; 6 Hinterwandinfarkte). Die Proben wurden in verschiedenen Intervallen 1 h - 35 h nach Schmerzbeginn abgenommen. 60 % der Proben wurden in den ersten 6 Stunden nach Schmerzbeginn abgenommen. Alle Patienten mit AMI wurden koronarangiographiert (9 Pat. akut und 1 Pat. im Verlauf). Bei 9 Patienten mit AMI wurden eine Reperfusionstherapie, d.h. Thrombolyse, primäre perkutane transluminale Koronarangioplastie (PTCA), Thrombolyse mit Rescue-PTCA oder akute aorto-koronare Venen-Bypass-Operation (ACVB-OP) durchgeführt. Bei einer Patientin zeigte sich in der akut durchgeführten Koronarangiographie ein spontane Rekanalisation im Infarktgefäß und ein Restthrombus mit gutem poststenotischen Fluß, so daß keine Primär-PTCA mehr durchgeführt wurde. In der Vergleichsgruppe wurde ein Patient mit heftigen akuten Thoraxschmerzen akut koronarangiographiert, wobei eine koronare Herzerkrankung ausgeschlossen werden konnte. In der Vergleichsgruppe bestanden verschiedene Erkrankungen wie instabile Angina pectoris, stabile Angina pectoris, Skelettmuskeltrauma, Myopathie, Z.n. Lungenembolie, Pleuritis und Niereninsuffizienz. Bei den Patienten mit akutem Myokardinfarkt zeigte sich ein typisches Muster an Komplikationen wie Linksherzinsuffizienz und Herzrhythmusstörungen. Ein Patient mit einem Vorderwandinfarkt kam in einen reanimationspflichtigen kardiogenen Schock, den er aber nach Thrombolyse, Akut-PTCA mit Stent-Implantation und intraaortaler Ballonpumpen- (IABP)-Implantation überlebte.

In allen Proben wurden eine Konzentrationsbestimmung von CCTD durchgeführt.

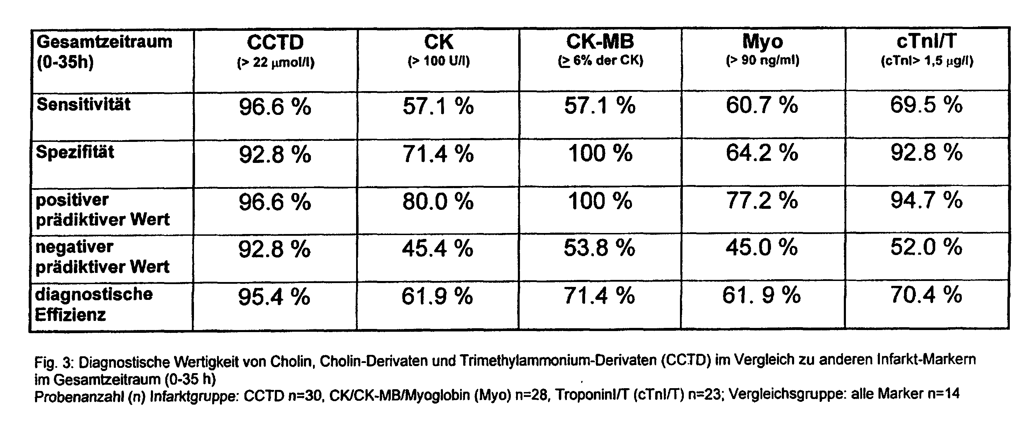

Die Bewertung der Meßergebnisse von CCTD erfolgte unter Berücksichtigung des ermittelten Grenzwertes. Wie bereits ausgeführt, zeigen Meßwerte oberhalb des Grenzwertes einen akuten Myokardinfarkt an; bei Meßwerten unterhalb des Grenzwertes liegt dagegen kein akuter Myokardinfarkt vor. Die Bestimmung der diagnostischen Wertigkeit wurde Proben-bezogen durchgeführt, d.h. die Bewertung des einzelnen Meßergebnisses wurde unabhängig von früheren oder späteren Meßergebnissen des Patienten vorgenommen. Bei 29 der 30 Infarkt-Proben waren die CCTD über den Grenzwert erhöht. Umgekehrt waren mit Ausnahme einer Probe die CCTD bei allen Patienten ohne Infarkt niedriger als der Grenzwert. Fig. 3 zeigt die Daten zur diagnostischen Wertigkeit von CCTD für die Diagnose eines akuten Myokardinfarktes für den Gesamtzeitraum von 0-35 h. Fig. 3 zeigt, daß CCTD mit 96.6 % die höchste Sensitivität und mit 95.4 % die höchste diagnostische Effizienz im Vergleich zu allen anderen Infarkt-Markem haben. Die hohe diagnostische Wertigkeit von CCTD ist auch deshalb bemerkenswert, weil viele diagnostisch schwierige

Patienten untersucht wurden, d.h. Patienten mit Mikro-Infarkten (4 von 10 Patienten), Patienten in den ersten Stunden des Infarktes und Patienten mit Skelettmuskeltrauma.

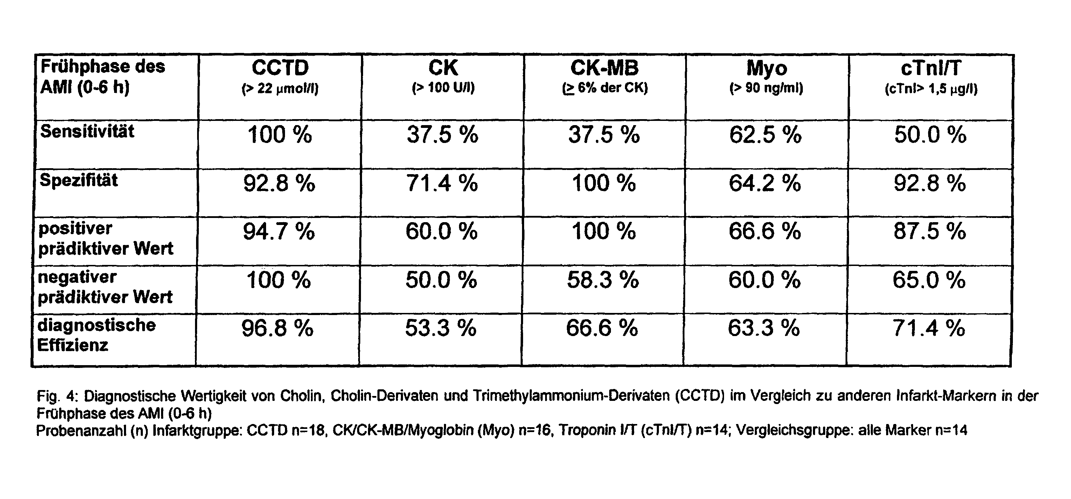

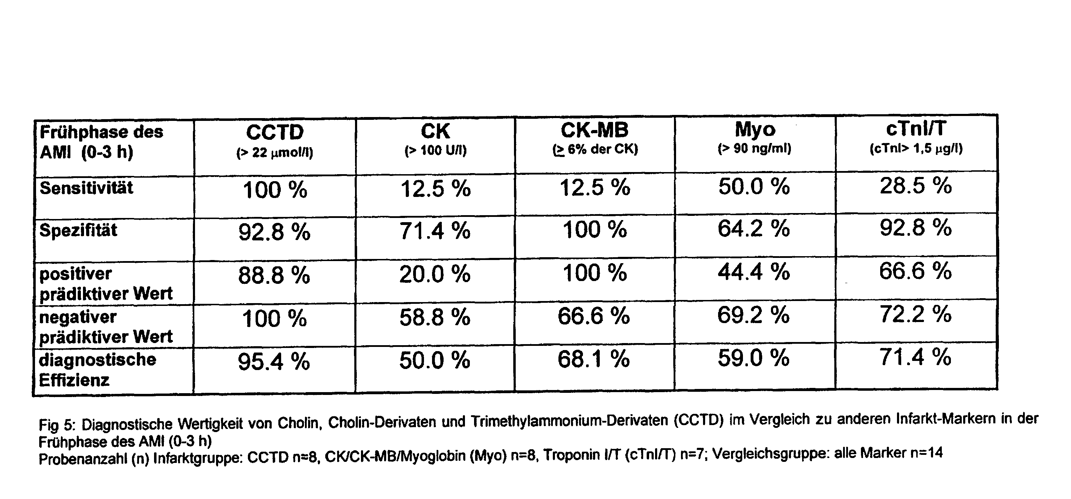

CCTD waren in ihrer diagnostischen Wertigkeit insbesondere in der Frühphase des AMI den konventionellen Infarkt-Markern überlegen. Alle Infarkt-Patienten in der Zeitspanne 0 bis 6 Stunden nach Schmerzbeginn waren CCTD-positiv, während nur 37.5 % der Patienten-Proben eine pathologische CK oder CK-MB hatten (siehe Fig. 4). Myoglobin hatte entsprechend seiner raschen Freisetzungskinetik die zweithöchste Sensitivität mit 62.5 %, ist aber, wie bekannt, nicht myokardspezifisch. In den ersten 6 h wiesen nur 50 % der Proben der Patienten mit AMI eine pathologische Troponin I/T-Konzentration auf.
Die Vorteile von CCTD kommen noch deutlicher zur Darstellung, wenn man lediglich Patienten-Proben in den Stunden 0-3 nach Schmerzbeginn betrachtet (Fig. 5). Während die konventionellen Infarkt-Marker in den ersten drei Stunden nach Schmerzbeginn kaum eine diagnostische Aussagekraft besitzen und eine diagnostische Effizienz zwischen 50% und 71% haben sind, erkennen CCTD alle untersuchten Infarkt-Proben in den ersten 3 h nach Schmerzbeginn. Weitere Untersuchungsergebnisse der Anmelder bestätigen die rasche Freisetzung von CCTD bei akuten koronaren Syndromen und ihre hohe diagnostische Aussagekraft in der Frühphase nach Beginn der Symptomatik. In der Spätphase des AMI kommt es zu einer geringen Verminderung der Sensitivität von CCTD.

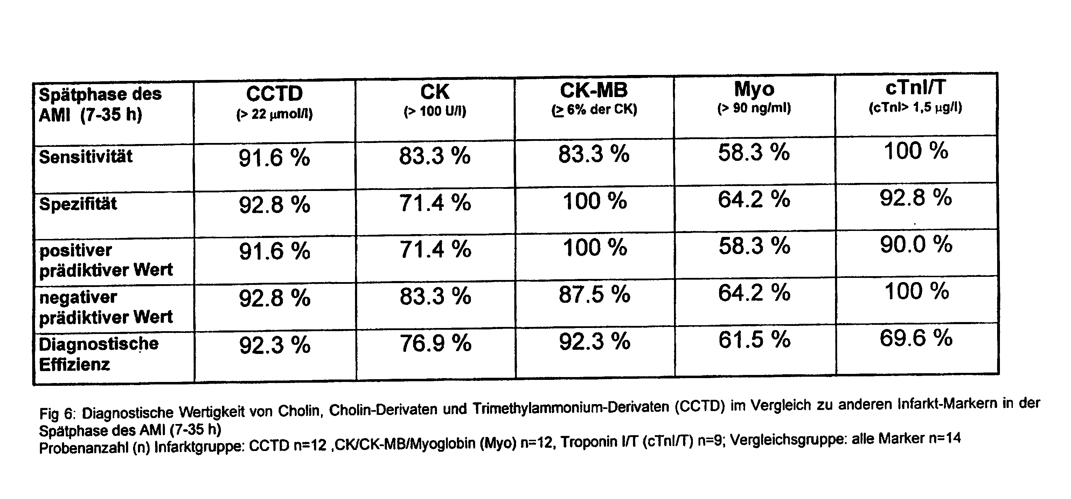

Fig. 6 zeigt die diagnostische Wertigkeit von CCTD in der Spätphase des AMI. In der Spätphase des AMI lag die Sensitivität von CCTD bei 91.6 %. Bei einer Probe kam es zu einem falsch-negativen Test-Ergebnis.
Eine Erhöhung von CCTD war zu 92.8 % spezifisch für einen akuten Myokardinfarkt. Bei einer Probe bestand ebenfalls eine Erhöhung von CCTD, ohne daß eine akuter Myokardinfarkt vorlag.

Die Auswertung von $^1$H-NMR-Spektren im Pattern-Recognition-Modus (gleichzeitige Bestimmung von CCTD, ihren Reaktionsprodukten, Creatin und Dimethylamin, sowie von beliebigen geeigneten Kombinationen davon) verbesserte die diagnostische Aussagekraft der Methode, so daß bei den bisher durchgeführten Untersuchungen alle Myokardinfarkte mit 100 % Sensitivität und 100 % Spezifität zu jedem Zeitpunkt diagnostiziert bzw. ausgeschlossen werden konnten.

Patienten mit instabiler Angina pectoris haben bekanntermaßen eine schlechtere Prognose, wenn im Verlauf erhöhte Werte kardialer Troponine festgestellt werden (Ohman E.M. 1996, N Engl J Med 335: 1333-41). Man nimmt an, daß diese Patienten nicht nur eine Myokardischämie durchmachen, sondern auch myokardiale Mikronekrosen erleiden, so daß nicht auszuschließen ist, daß diese Patienten in Zukunft bei neuen Klassifikationen des AMI als Mikroinfarkte klassifiziert werden. Die rasche Identifizierung solcher Patienten ist wichtig, da sie in der Regel ohne Zeitverzug therapiert werden sollten und gegebenenfalls notfallmäßig angiographiert werden müssen. Die Untersuchungen der Anmelder zeigten, daß die Patienten mit Angina pectoris und unkompliziertem Verlauf keine erhöhten CCTD haben, hingegen alle Patienten mit myokardialen Mikronekrosen erhöhte CCTD-Werte aufweisen. Nach den bisherigen Ergebnissen entwickeln alle Patienten mit akuten koronaren Syndromen, die CCTD-positiv sind, im Verlauf erhöhte Troponin-Werte. Die seriellen Untersuchungen zeigten ebenfalls, daß bei Patienten mit Mikronekrosen die CCTD um mehrere Stunden früher positiv waren als Troponin I oder Troponin T. Des weiteren ist davon auszugehen, daß das Verfahren ebenfalls hilfreich bei der Diagnostik schwerer myokardialer Ischämien, z.B. bei Katheterinterventionen an den Koronarien, oder bei Erkrankungen mit Beteiligung des Myokards, z.B. bei Myokarditiden, ist. Die Ergebnisse der Anmelder belegen, daß das Verfahren sowohl beim akuten Myokardinfarkt als auch bei der instabilen Angina pectoris, d.h. allgemein bei Verdacht auf ein akutes koronares Syndrom, wertvoll ist.

Da freigesetzte CCTD durch verschiedene biochemische Prozesse weiter metabolisiert bzw. modifiziert werden, entstehen neben Cholin, Cholin-Derivaten und Trimethylammonium-Derivaten auch vermehrt Reaktionsprodukte von CCTD. Diese Reaktionsprodukte können Bruchstücke oder bestimmte Metabolite von CCTD darstellen. Wie z.B. von den CK-Isoenzymen bekannt, kann die gleichzeitige Bestimmung solcher Reaktionsprodukte von Infarkt-Markem diagnostisch vorteilhaft sein. Die Untersuchungen der Anmelder zeigten bei einem Teil der Infarkt-Patienten Hinweise auf die Entstehung solcher Reaktionsprodukte von CCTD. Die Entstehung der Reaktionsprodukte geht u.a. auf die gesteigerte Aktivität bestimmter Phospholipasen zurück. Neben der Phosholipase $A_2$, die u.a. zur Freisetzung von dem genannten Lysoplasmenylcholin beiträgt, sind, wie anfangs erwähnt, weitere wichtige Enzyme dieser Gruppe die Phospholipasen C und D. Reaktionsprodukte der Aktivität dieser beiden Phospholipasen in Einwirkung auf die Plasmalogene sind z.B. 1-O-Alk-1'-enyl-2-substituiertes Glycerol (durch Phospholipase C) bzw. 1-O-Alk-1'-enyl-2-substituiertes Glycerolphosphat (durch Phospholipase D). Diese Reaktionsprodukte werden gemeinsam mit Cholin bzw. Phosphorylcholin vermehrt gebildet, wenn bei Myokardischämie die genannten Phospholipasen auf Plasmenylcholine einwirken. Das Verfahren ist also auch durch den Nachweis der genannten einfachen Reaktionsprodukte von CCTD, gegebenenfalls in Kombination mit CCTD-Bestimmungen möglich.

**Zeitverlauf der Freisetzung von CCTD beim akuten Myokardinfarkt**

Die Analyse aller verfügbaren Meßwerte für CCTD (mit Ausschluß einer nicht-repräsentativen Probe) zeigte, daß CCTD innerhalb von 60 min nach Schmerzbeginn positiv werden und offenbar eine biphasische Freisetzungskinetik mit einem frühen Maximum nach 2-3-h und einem zweiten Maximum nach 5-6 h haben. Der Vergleich mit anderen Infarkt-Markern zeigte, daß die Freisetzungskinetik von CCTD entsprechend dem niedrigen Molekulargewicht erheblich schneller ist als die Freisetzung von den bisher bekannten Markern, was die hohe diagnostische Wertigkeit in der Frühphase erklärt.
Weitere von den Anmeldern durchgeführte Untersuchungen bei über 100 Patienten mit Brustschmerzen konnten die Überlegenheit des erfindungsgemäßen Verfahrens gegenüber konventionellen Markern sowie die erfindungsgemäßen Eigenschaften für die Frühdiagnostik von akuten koronaren Syndromen bestätigen.
Das Verfahren zur Erkennung des akuten Myokardinfarktes und schwerer Formen der instabilen Angina pectoris durch die Bestimmung und Bewertung des Gehaltes von Cholin, Cholin- und/oder Trimethylammonium-Derivaten, ausgewählt aus der Gruppe, die Phosphorylcholin, Plasmalogene und Lysoplasmenylcholin umfaßt, und deren einfachen Reaktionsprodukten in Körperflüssigkeiten scheint allen bisher eingeführten nicht-invasiven Verfahren, einschließlich der Bestimmung bekannter biochemischer Marker zur Diagnostik akuter koronarer Syndrome, überlegen zu sein. Neben dem akuten Myokardinfarkt können ebenfalls schwere Formen der instabilen Angina pectoris mit myozytären Nekrosen, welche im Verlauf erhöhte Troponin-Werte aufweisen, durch das Verfahren fiühzeitiger erkannt werden. Diese Ergebnisse belegen, daß das Verfahren sowohl beim akuten Myokardinfarkt als auch bei der instabilen Angina pectoris, d.h. allgemein bei Verdacht auf ein akutes koronares Syndrom, wertvoll ist. Der besondere Wert der Erfindung wird auch dadurch unterstrichen, daß es mit dem erfindungsgemäßen in vitro Verfahren erstmals möglich sein wird, Patienten mit akuten koronaren Syndromen und akutem Myokardinfarkt frühzeitig, d.h. in den ersten drei Stunden nach Schmerzbeginn, sicher zu diagnostizieren bzw. eine Ausschlußdiagnose zu stellen. Dies ist durch kein publiziertes in vitro Verfahren mit einer vergleichbaren hohen Sicherheit möglich. Die American Heart Association und das American College of Cardiology stellen fest, daß es einen " eindeutigen Bedarf für bessere Methoden zur prompten Identifikation von Patienten mit akutem Myokardinfarkt gibt, die so genau und so früh wie möglich anwendbar sein müssen" (übersetzt aus Ryan, T.J. et al. ACC/AHA Guidelines for the Management of Patients with Acute Myocardial Infarction, 1996, J Am Coll Cardiol 28: 1328-1428, S. 1340). Eine solche Methode gibt es zur Zeit nicht, und nach den den Anmeldern vorliegenden Daten erfüllt das erfindungsgemäße Verfahren genau die geforderten Eigenschaften.

Das Verfahren bezieht sich auf die Diagnostik der klar definierten Krankheitsbilder akuter koronarer Syndrome, die die instabile Angina pectoris und den akuten Myokardinfarkt umfassen, der als Q-wave- oder als non-Q-wave Myokardinfarkt verlaufen kann. Untersuchungen zu anderen Krankheitsbildern, wie z.B. dem Hirninfarkt, die hinsichtlich ihrer Pathomorphologie und Pathobiochemie in vielerlei Hinsicht unterschiedlich sind, erlauben keine Analogieschlüsse in Bezug auf das erfindungsgemäße Verfahren.

Die Durchführung des erfindungsgemäßen Verfahrens zur Frühdiagnostik akuter koronarer Syndrome ist mit verschiedenen Bestimmungsmethoden bzw. Techniken möglich, zumal gleichwertige Meßergebnisse bestimmter Substanzen häufig mit unterschiedlichen Meßmethoden zu erheben sind, die im Fall von CCTD vielfältig in der Literatur beschrieben worden sind. Das Verfahren umfaßt neben der Auswahl *einer* geeigneten Meßmethode, die Auswahl *einer* geeigneten Körperflüssigkeit, die verfahrens- und methodengerechte Probenvorbereitung, die spezifische Messung des Gehaltes von CCTD und/oder deren Reaktionsprodukten, ausgewählt aus der Gruppe, die 1-O-Alk-1'-enyl-2-substituiertes Glycerol und 1-O-Alk-1'-enyl-2-substituiertes Glycerolphosphat umfaßt, und die verfahrensgerechte Auswertung zur Diagnostik von Patienten mit akuten koronaren Syndromen. Da die Freisetzung von CCTD in verschiedene andere Körperflüssigkeiten und Körperbestandteile erfolgt, ist die Anwendung des Verfahrens auch bei der Untersuchung anderer Körperbestandteile, z.B. Gewebeproben, möglich. Dabei ist allerdings die diagnostische Wertigkeit im Vergleich zu dem beschriebenen Anwendungsbeispiel möglicherweise vermindert. Da im Rahmen der Freisetzung von CCTD auch vermehrt Reaktionsprodukte der CCTD, ausgewählt aus der Gruppe, die 1-O-Alk-1'-enyl-2-substituiertes Glycerol und 1 -O-Alk-1'-enyl-2-substituiertes Glycerolphosphat umfaßt, anfallen, ist das Verfahren somit auch durch den Nachweis solcher Reaktionsprodukte möglich. Des weiteren ist es möglich, daß das Verfahren so durchgeführt wird, daß semi-quantitative oder qualitative Aussagen getroffen werden, d.h. daß z.B. Schnell-Tests für CCTD durch eine Farbreaktion lediglich anzeigen, ob ein Myokardinfarkt vorliegt oder nicht. Das Verfahren kann so durchgeführt werden, daß Zustände oder Vorgänge beobachtet oder hervorgerufen werden, die durch den Gehalt von CCTD oder CCTD-Reaktionsprodukten determiniert werden. Neben der Erkennung von akuten koronaren Syndromen bietet das Verfahren Aussicht auf weitere diagnostische Informationen wie Infarktgröße, Prognose, Therapiekontrolle und der Vorhersage bestimmter Komplikationen, Risiken und des klinischen Verlaufs. Der besondere Wert des erfindungsgemäßen Verfahrens wird dadurch hervorgehoben, daß es die diagnostischen Eigenschaften aufweist, die von internationalen Expertenkommissionen, wie der American Heart Association und dem American College of Cardiology für in vitro Verfahren zur Frühdiagnostik von akuten koronaren Syndromen seit langem gefordert werden.

Insgesamt soll das Verfahren helfen, die erheblichen Probleme der derzeitigen Frühdiagnostik akuter koronarer Syndrome zu vermindern und die Diagnostik und Therapie der erkrankten Patienten zu verbessern.

Die in der vorstehenden Beschreibung, den Ansprüchen und der Zeichnung offenbarten Merkmale können sowohl einzeln als auch in beliebiger Kombination für die Verwirklichung der Erfindung von Bedeutung sein.

## Patentansprüche

1. In vitro Verfahren zur Erkennung und Diagnostik akuter koronarer Syndrome, insbesondere des akuten Myokardinfarktes, **dadurch gekennzeichnet, daß** der Gehalt von Cholin, Cholin- und/oder Trimethylammonium-Derivaten, ausgewählt aus der Gruppe, die aus Phosphorylcholin, Plasmologenen und Lysoplasmenylcholin besteht, in Proben von Körperflüssigkeiten oder Proben von Körperbestandteilen bestimmt wird.

2. In vitro Verfahren nach Anspruch 1, **dadurch gekennzeichnet, daß** der Gehalt von Cholin, Cholin- und/oder Trimethylammonium-Derivaten, ausgewählt aus der Gruppe, die aus Phosphorylcholin, Plasmologenen und Lysoplasmenylcholin besteht, unter Berücksichtigung eines Grenzwertes bewertet wird.

3. In vitro Verfahren zur Erkennung und Diagnostik akuter koronarer Syndrome, insbesondere des akuten Myokardinfarktes, **dadurch gekennzeichnet, daß** der Gehalt von Reaktionsprodukten von Cholin, Cholin- und/oder Trimethylammonium-Derivaten, ausgewählt aus der Gruppe, die aus Phosphorylcholin, Plasmalogenen und Lysoplasmenylcholin besteht, in Proben von Körperflüssigkeiten oder Proben von Körperbestandteilen bestimmt wird, wobei die Reaktionsprodukte ausgewählt sind aus der Gruppe, die aus 1-O-Alk-1'-enyl-2-substituierten Glycerol und 1-O-Alk-1'-enyl-2-substituierten Glycerolphosphat besteht.

4. In vitro Verfahren zur Erkennung und Diagnostik akuter koronarer Syndrome, insbesondere des akuten Myokardinfarktes, **dadurch gekennzeichnet, daß** in Proben von Körpertlüssigkeiten oder Proben von Körperbestandteilen quantitative, semi-quantitative oder qualitative Beobachtungen gemacht werden, die durch den Gehalt von Cholin, Cholin- und/oder Trimethylammonium-Derivaten, ausgewählt aus der Gruppe, die aus Phosphorylcholin, Plasmalogenen und Lysoplasmenylcholin besteht, und/oder ihren Reaktionsprodukten, ausgewählt aus der Gruppe, die aus 1-O-Alk-1'-enyl-2-substituierten Glycerol und 1-O-Alk-1'-enyl-2-substituierten Glycerolphosphat besteht bestimmt werden.

5. In vitro Verfahren nach einem der vorangehenden Ansprüche, **dadurch gekennzeichnet, daß** zur Bestimmung von Cholin, Cholin- und/oder Trimethylammonium-Derivaten, ausaus der Gruppe, die aus Phosphorylcholin, Plasmalogenen und Lysoplasmenylcholin besteht, und/oder ihren Reaktionsprodukten, ausgewählt aus der Gruppe, die aus 1-O-Alk-1'-enyl-2-substituierten Glycerol und 1-O-Alk-1'-enyl-2-substituierten Glycerolphosphat besteht Kernspinresonanz(NMR)-Methoden, enzymatische, immunologische, chromatographische, massenspektrometrische, elektrochemische, photometrische Methoden oder Methoden eingesetzt werden, bei denen durch Hinzugabe von Reagenzien (z.B. Enzymen) chemische Reaktionen bzw. Reaktionsprodukte erzeugt werden, die dann die Detektion und Messung der Gesamtgruppe, einer Subgruppe oder Subspezies der CCTD ermöglichen.

6. In vitro Verfahren zur Erkennung und Diagnostik akuter koronarer Syndrome, insbesondere des akuten Myokardinfarktes, **dadurch gekennzeichnet, daß** eine NMR-Spektroskopie von Proben von Körperflüssigkeiten oder Proben von Körperbestandteilen durchgeführt wird und die Auswertung durch eine "Mustererkennung" (Pattern-Recognition) mehrerer Substanzen, umfassend Cholin, Cholin- und/oder Trimethylammonium-Derivate, ausgewählt aus der Gruppe, die aus Phosphorylcholin, Plasmalogenen und Lysoplasmenylcholin besteht die Reaktionsprodukte, ausgewählt aus der Gruppe, die aus 1-O-Alk-1'-enyl-2-substituierten Glycerol und 1-O-Alk-1'-enyl-2-substituierten Glycerolphosphat besteht, und Creatin und Dimethylamin, vorgenommen wird.

7. In vitro Verfahren nach einem der vorangehenden Ansprüche, **dadurch gekennzeichnet, daß** eine Bestimmung von Cholin, Cholin- und/oder Trimethylammonium-Derivaten, ausgewählt aus der Gruppe, die aus Phosphorylcholin, Plasmalogenen und Lysoplasmenylcholin besteht und/oder ihren Reaktionsprodukten, ausgewählt aus der Gruppe, die aus 1-O-Alk-1'-enyl-2-substituierten Glycerol und 1-O-Alk-1'-enyl-2-substituierten Glycerolphosphat besteht vorgenommen wird in einer Probe von Körperflüssigkeit, wobei die Körperflüssigkeit ausgewählt ist aus einer Gruppe, die Serum, Plasma, Vollblut, aufbereitete Blutproben und Harn umfaßt.

## Claims

1. An in vitro method of recognizing and diagnosing acute coronary syndroms, especially an acute myocardial infarction, **characterized by** determining the content of choline, choline and/or trimethyl ammonium derivatives selected from the group consisting of phosphoryl choline, plasmalogens, and lysoplasmenyl choline, in samples of body fluids or samples of component parts of the body.

2. The in vitro method as claimed in claim 1, **characterized in that** the content of choline, choline and/or trimethyl ammonium derivatives selected from the group consisting of phosphoryl choline, plasmalogens, and lysoplasmenyl choline is evaluated taking into account a limit value.

3. An in vitro method of recognizing and diagnosing acute coronary syndroms, especially an acute myocardial infarction, **characterized by** determining the content of reaction products of choline, choline and/or trimethyl ammonium derivatives selected from the group consisting of phosphoryl choline, plasmalogens, and lysoplasmenyl choline, in samples of body fluids or samples of component parts of the body, the reaction products being selected from the group consisting of 1-O-alk-1'-enyl-2 substituted glycerol and 1-O-alk-1'-enyl-2 substituted glycerol phosphate.

4. An in vitro method of recognizing and diagnosing acute coronary syndroms, especially an acute myocardial infarction, **characterized in that** quantitative, semiquantitative, or qualitative observations are made in samples of body fluids or samples of component parts of the body, said observations being determined by the content of choline, choline and/or trimethyl ammonium derivatives selected from the group consisting of phosphoryl choline, plasmalogens, and lysoplasmenyl choline, and/or the reaction products thereof selected from the group consisting of 1-O-alk-1'-enyl-2 substituted glycerol and 1-O-alk-1'-enyl-2 substituted glycerol phosphate.

5. The in vitro method as claimed in any one of the preceding claims, **characterized in that** nuclear magnetic resonance (NMR) methods, enzymatic, immunological, chromatographic, mass spectrometric, electrochemical, photometric methods or methods are applied in which methods chemical reactions and reaction products respectively are generated by the addition of reagents (e. g. enzymes), said chemical reactions and reaction products allowing for the detection and measuring of the whole group, a subgroup or subspecies of CCTD, to determine choline, choline and/or trimethyl ammonium derivatives selected from the group consisting of phosphoryl choline, plasmalogens, and lysoplasmenyl choline, and/or the reaction products thereof selected from the group consisting of 1-O-alk-1'-enyl-2 substituted glycerol and 1-O-alk-1'-enyl-2 substituted glycerol phosphate.

6. An in vitro method of recognizing and diagnosing acute coronary syndroms, especially an acute myocardial infarction, **characterized in that** samples of body fluids or samples of component parts of the body are subjected to NMR spectroscopy and the evaluation is accomplished by pattern recognition of a plurality of substances consisting of choline, choline and/or trimethyl ammonium derivatives selected from the group consisting of phosphoryl choline, plasmalogens, and lysoplasmenyl choline, of the reaction products thereof selected from the group consisting of 1-O-alk-1'-enyl-2 substituted glycerol and 1-O-alk-1'-enyl-2 substituted glycerol phosphate, and of creatine and dimethyl amine.

7. The in vitro method as claimed in any one of the preceding claims, **characterized by** determining choline, choline and/or trimethyl ammonium derivatives selected from the group consisting of phosphoryl choline, plasmalogens, and lysoplasmenyl choline, and/or the reaction products thereof selected from the group consisting of 1-O-alk-1'-enyl-2 substituted glycerol and 1-O-alk-1'-enyl-2 substituted glycerol phosphate, in a sample of body fluid selected from a group consisting of serum, plasma, whole blood, prepared blood samples, and urine.

## Revendications

1. Procédé in vitro pour la détection et le diagnostic de syndromes coronariens aigus, en particulier de l'infarctus du myocarde aigu, **caractérisé par le fait que** la teneur en choline, en dérivés de choline et/ou de triméthylammonium, qui sont choisis dans le groupe constitué par la phosphorylcholine, le plasmalogène et la lysoplasménylcholine, est déterminée dans des prélèvements de fluides corporels ou de constituants corporels.

2. Procédé in vitro selon la revendication 1, **caractérisé par le fait que** la teneur en choline, en dérivés de choline et/ou de triméthylammonium, qui sont choisis dans le groupe constitué par la phosphorylcholine, le plasmalogène et la lysoplasménylcholine, est déterminée en tenant compte d'une valeur limite.

3. Procédé in vitro pour la détection et le diagnostic de syndromes coronariens aigus, en particulier de l'infarctus du myocarde aigu, **caractérisé par le fait que** la teneur en choline, en dérivés de choline et/ou de triméthylammonium, qui sont choisis dans le groupe constitué par la phosphorylcholine, le plasmalogène et la lysoplasménylcholine, est déterminée dans des prélèvements de fluides corporels ou de constituants corporels, les produits réactionnels étant choisis dans le groupe constitué par les glycérols 1-O-alc-1'-ényl-2-substitués et les glycérophosphates 1-O-alc-1'-ényl-2-substitués.

**4.** Procédé in vitro pour la détection et le diagnostic de syndromes coronariens aigus, en particulier de l'infarctus du myocarde aigu, **caractérisé par le fait que** l'on effectue, dans des prélèvements de fluides corporels ou de constituants corporels, des déterminations quantitatives, servi-quantitative: ou qualitatives de la teneur en choline, en dérivés de choline et/ou de triméthylammonium, choisis dans le groupe constitué par la phosphorylcholine, le plasmalogène et la lysoplasménylcholine, et/ou de la teneur en leurs produits réactionnels choisis dans le groupe constitué par les glycérols 1-O-alc-1'-ényl-2-substitués et les glycérophosphates 1-O-alc-1'-ényl-2-substitués.

**5.** Procédé in vitro selon l'une quelconque des revendications précédentes, **caractérisé par le fait que** pour la détermination de la teneur en choline, en dérivés de choline et/ou de triméthylammonium, choisis dans le groupe constitué par la phosphorylcholine, le plasmalogène et la lysoplasménylcholine et/ou de la teneur en leurs produits réactionnels choisis dans le groupe constitué par les glycérols 1-O-alc-1'-ényl-2-substitués et les glycérophosphates 1-O-alc-1'-ényl-2-substitués, on applique des méthodes de résonance magnétique nucléaire (RMN), des méthodes enzymatiques, immunologiques, chromatographiques, de spectrométrie de masse, électrochimiques, photométriques ou des méthodes dans lesquelles par addition de réactants (par exemple, des enzymes), on obtient des réactions chimiques ou des produits réactionnels qui permettent ensuite d'effectuer la détection et la mesure du groupe CCTD (choline, dérivés de choline et/ou de triméthylammonium) entier, d'un sous-groupe ou d'une sous-espèce de celui-ci.

**6.** Procédé in vitro pour la détection et le diagnostic de syndromes coronariens aigus, en particulier de l'infarctus du myocarde aigu, **caractérisé par le fait que** l'on effectue une spectroscopie RMN sur des prélèvements de fluides corporels ou de constituants corporels et que l'on procède à l'évaluation des résultats à l'aide d'une méthode de reconnaissance de modèle (Pattern Récognition) de plusieurs substances comprenant la choline, les dérivés de choline et/ou de triméthylammonium, choisis dans le groupe constitué par la phosphorylcholine, le plasmalogène et la lysoplasménylcholine, leurs produits réactionnels choisis dans le groupe constitué par les glycérols 1-O-alc-1'-ényl-2-substitués et les glycérophosphates 1-O-alc-1'-ényl- substitués, ainsi que la créatinine et la diméthylamine.

**7.** Procédé in vitro selon l'une quelconque des revendications précédentes, **caractérisé par le fait que** la teneur en choline, en dérivés de choline et/ou de triméthylammonium, choisis dans le groupe constitué par la phosphorylcholine, le plasmalogène et la lysoplasménylcholine, et/ou la teneur en leurs produits réactionnels choisis dans le groupe constitué par les glycérols 1-O-alc-1'-ényl-2-substitués et les glycérophosphates 1-O-alc-1'-ényl-2-substitués, est déterminée dans un prélèvement de fluide corporel, le fluide corporel étant choisi dans le groupe constitué par le sérum, le plasma, le sang total, des préparations à base d'échantillons de sang et les urines.

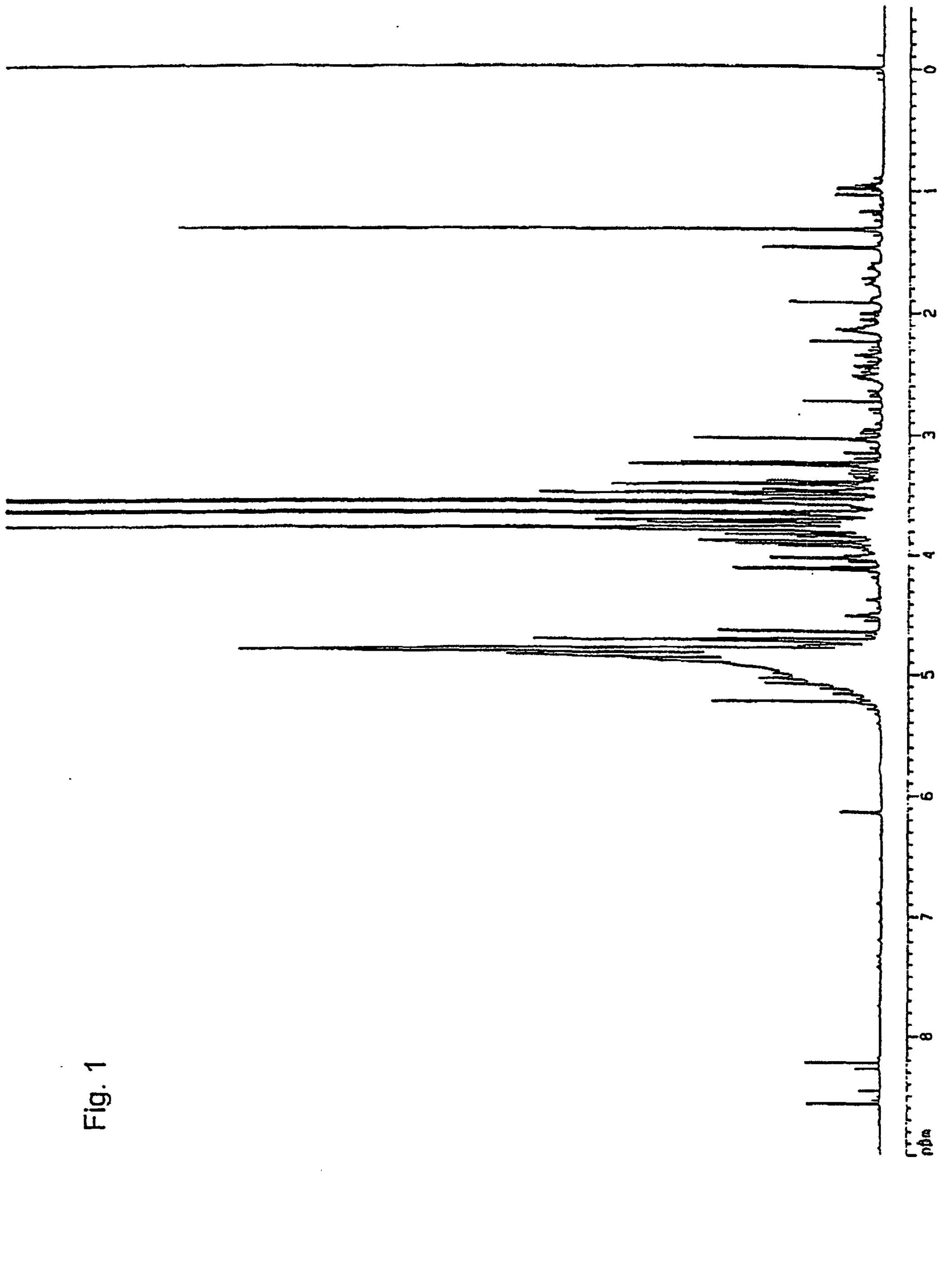


Fig. 1

Fig. 2

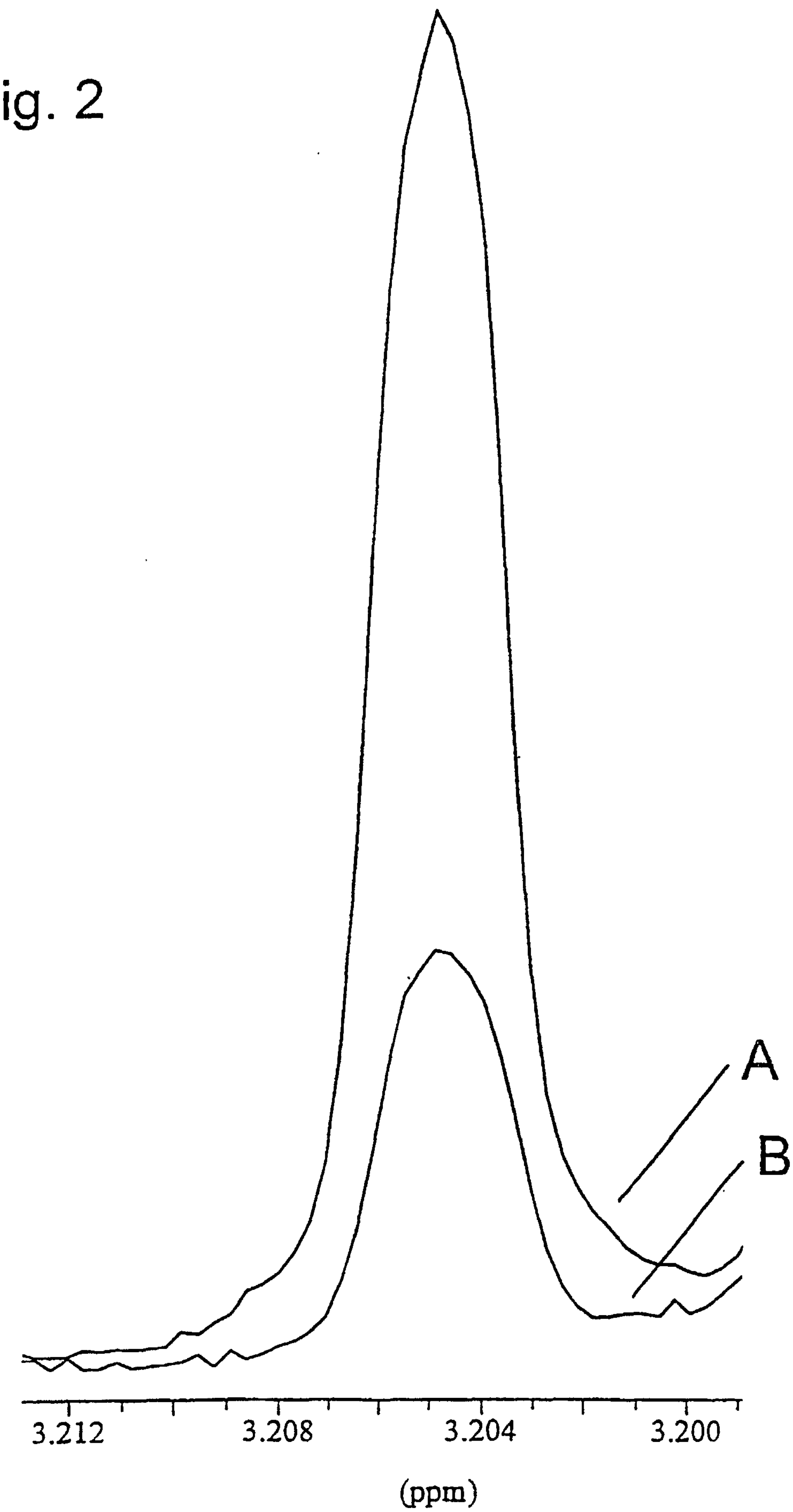

| Gesamtzeitraum (0-35h) | CCTD (> 22 μmol/l) | CK (> 100 U/l) | CK-MB (≥ 6% der CK) | Myo (> 90 ng/ml) | cTnI/T (cTnI> 1,5 μg/l) |
|---|---|---|---|---|---|
| Sensitivität | 96.6 % | 57.1 % | 57.1 % | 60.7 % | 69.5 % |
| Spezifität | 92.8 % | 71.4 % | 100 % | 64.2 % | 92.8 % |
| positiver prädiktiver Wert | 96.6 % | 80.0 % | 100 % | 77.2 % | 94.7 % |
| negativer prädiktiver Wert | 92.8 % | 45.4 % | 53.8 % | 45.0 % | 52.0 % |
| diagnostische Effizienz | 95.4 % | 61.9 % | 71.4 % | 61. 9 % | 70.4 % |

Fig. 3: Diagnostische Wertigkeit von Cholin, Cholin-Derivaten und Trimethylammonium-Derivaten (CCTD) im Vergleich zu anderen Infarkt-Markern im Gesamtzeitraum (0-35 h)
Probenanzahl (*n*) Infarktgruppe: CCTD n=30, CK/CK-MB/Myoglobin (Myo) n=28, TroponinI/T (cTnI/T) n=23; Vergleichsgruppe: alle Marker n=14

| Frühphase des AMI (0-6 h) | CCTD (> 22 µmol/l) | CK (> 100 U/l) | CK-MB (≥ 6% der CK) | Myo (> 90 ng/ml) | cTnI/T (cTnI> 1,5 µg/l) |
|---|---|---|---|---|---|
| Sensitivität | 100 % | 37.5 % | 37.5 % | 62.5 % | 50.0 % |
| Spezifität | 92.8 % | 71.4 % | 100 % | 64.2 % | 92.8 % |
| positiver prädiktiver Wert | 94.7 % | 60.0 % | 100 % | 66.6 % | 87.5 % |
| negativer prädiktiver Wert | 100 % | 50.0 % | 58.3 % | 60.0 % | 65.0 % |
| diagnostische Effizienz | 96.8 % | 53.3 % | 66.6 % | 63.3 % | 71.4 % |

Fig. 4: Diagnostische Wertigkeit von Cholin, Cholin-Derivaten und Trimethylammonium-Derivaten (CCTD) im Vergleich zu anderen Infarkt-Markern in der Frühphase des AMI (0-6 h)
Probenanzahl (n) Infarktgruppe: CCTD n=18, CK/CK-MB/Myoglobin (Myo) n=16, Troponin I/T (cTnI/T) n=14; Vergleichsgruppe: alle Marker n=14

| Frühphase des AMI (0-3 h) | CCTD (> 22 µmol/l) | CK (> 100 U/l) | CK-MB (≥ 6% der CK) | Myo (> 90 ng/ml) | cTnI/T (cTnI> 1,5 µg/l) |
|---|---|---|---|---|---|
| Sensitivität | 100 % | 12.5 % | 12.5 % | 50.0 % | 28.5 % |
| Spezifität | 92.8 % | 71.4 % | 100 % | 64.2 % | 92.8 % |
| positiver prädiktiver Wert | 88.8 % | 20.0 % | 100 % | 44.4 % | 66.6 % |
| negativer prädiktiver Wert | 100 % | 58.8 % | 66.6 % | 69.2 % | 72.2 % |
| diagnostische Effizienz | 95.4 % | 50.0 % | 68.1 % | 59.0 % | 71.4 % |

Fig 5: Diagnostische Wertigkeit von Cholin, Cholin-Derivaten und Trimethylammonium-Derivaten (CCTD) im Vergleich zu anderen Infarkt-Markern in der Frühphase des AMI (0-3 h)
Probenanzahl (n) Infarktgruppe: CCTD n=8, CK/CK-MB/Myoglobin (Myo) n=8, Troponin I/T (cTnI/T) n=7; Vergleichsgruppe: alle Marker n=14

| Spätphase des AMI (7-35 h) | CCTD (> 22 µmol/l) | CK (> 100 U/l) | CK-MB (≥ 6% der CK) | Myo (> 90 ng/ml) | cTnI/T (cTnI> 1,5 µg/l) |
|---|---|---|---|---|---|
| Sensitivität | 91.6 % | 83.3 % | 83.3 % | 58.3 % | 100 % |
| Spezifität | 92.8 % | 71.4 % | 100 % | 64.2 % | 92.8 % |
| positiver prädiktiver Wert | 91.6 % | 71.4 % | 100 % | 58.3 % | 90.0 % |
| negativer prädiktiver Wert | 92.8 % | 83.3 % | 87.5 % | 64.2 % | 100 % |
| Diagnostische Effizienz | 92.3 % | 76.9 % | 92.3 % | 61.5 % | 69.6 % |

Fig 6: Diagnostische Wertigkeit von Cholin, Cholin-Derivaten und Trimethylammonium-Derivaten (CCTD) im Vergleich zu anderen Infarkt-Markern in der Spätphase des AMI (7-35 h)
Probenanzahl (n) Infarktgruppe: CCTD n=12 ,CK/CK-MB/Myoglobin (Myo) n=12, Troponin I/T (cTnI/T) n=9; Vergleichsgruppe: alle Marker n=14